(19) Europäisches Patentamt / European Patent Office / Office européen des brevets

(11) EP 3 415 911 B1

(12) # EUROPEAN PATENT SPECIFICATION

(45) Date of publication and mention of the grant of the patent:
**14.12.2022 Bulletin 2022/50**

(21) Application number: **17750217.6**

(22) Date of filing: **06.02.2017**

(51) International Patent Classification (IPC):
***G01N 21/77*** *(2006.01)* *G01N 21/47* *(2006.01)*
*B01J 19/00* *(2006.01)*

(52) Cooperative Patent Classification (CPC):
**G01N 21/7743; G01N 33/483; G01N 33/543;**
B01J 2219/00637; B01J 2219/00653;
B01J 2219/00702; B01J 2219/00725;
B01J 2219/00729; G01N 21/4788; G01N 2021/7773

(86) International application number:
**PCT/JP2017/004249**

(87) International publication number:
**WO 2017/138494 (17.08.2017 Gazette 2017/33)**

(54) **ANALYSIS PLATE, ANALYSIS METHOD, AND METHOD OF MANUFACTURING ANALYSIS PLATE**

ANALYSEPLATTE, ANALYSEVERFAHREN UND VERFAHREN ZUR HERSTELLUNG EINER ANALYSEPLATTE

PLAQUE D'ANALYSE, PROCÉDÉ D'ANALYSE, ET PROCÉDÉ DE FABRICATION DE PLAQUE D'ANALYSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.02.2016 JP 2016022179**

(43) Date of publication of application:
**19.12.2018 Bulletin 2018/51**

(73) Proprietors:
- **Zeon Corporation**
  **Tokyo 100-8246 (JP)**
- **Osaka Prefecture University Public Corporation**
  **Osaka 599-8531 (JP)**

(72) Inventors:
- **ENDO, Tatsuro**
  **Sakai-shi**
  **Osaka 599-8531 (JP)**
- **YAKABE, Hiroshi**
  **Tokyo 100-8246 (JP)**

(74) Representative: **Maiwald GmbH**
**Elisenhof**
**Elisenstraße 3**
**80335 München (DE)**

(56) References cited:

| | |
|---|---|
| **JP-A- 2005 300 467** | **JP-A- 2005 300 467** |
| **US-A1- 2003 017 580** | **US-A1- 2006 180 750** |
| **US-A1- 2009 001 284** | **US-A1- 2012 288 951** |

- **SHINPEI KADO ET AL: "Potassium-ion-selective sensing based on selective reflection of cholesteric liquid crystal membranes", JOURNAL OF INCLUSION PHENOMENA AND MACROCYCLIC CHEMISTRY, KLUWER ACADEMIC PUBLISHERS, DO, vol. 72, no. 1 - 2, 12 May 2011 (2011-05-12), pages 227-232, XP035009951, ISSN: 1573-1111, DOI: 10.1007/S10847-011-9970-1**
- **ENDO, TATSURO et al.: "Reflectometric detection of influenza virus in human saliva using nanoimprint lithography-based flexible two-dimensional photonic crystal biosensor", Sensors and Actuators B: Chemical, vol. 148, 2010, pages 269-276, XP029821199,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 415 911 B1



(Cont. next page)

- KOOY, NAZRIN et al.: "A Review of Roll-to-roll Nanoimprint Lithography", Nanoscale Research Letters, vol. 9, no. 320, 2014, pages 1-13, XP055209284,
- ENDO, TATSURO et al.: "Printed Two-dimensional Photonic Crystals for Single-step Label-free Biosensing of Insulin under Wet Conditions", Lab on a Chip, vol. 12, 2012, pages 1995-1999, XP055533194,
- TATSURO ENDO: "Development of Nanophotonics- based Bioanalytical Devices", Journal of Japan Society for Analytical Chemistry, vol. 64, no. 10, 2015, pages 751-757, XP055574421,
- JUNJI WATANABE: "Beautiful Structural Colors in Beetles Based on Selective Reflection of Light from Cholesteric Helicoidal Structure", Japanese Journal of Optics, vol. 33, no. 4, 2004, pages 34-40, XP009513029, ISSN: 0389-6625
- TATSURO ENDO et al.: "Fabrication of Optical Devices Based on Printable Photonics Technology and Its Application for Biosensor", The Transactions of the Institute of Electrical Engineers of Japan Sensor-Micromachine Bumonshi, vol. 130, no. 9, 2010, pages 450-451, XP009512216, ISSN: 1341-8939, DOI: 10.1541/ieejsmas.130.450

## Description

Field

The present invention relates to an analysis plate and an analysis method which can be used for analysis in various use applications, such as medical diagnosis, and a method for producing the analysis plate.

Background

Various methods are known in the prior art as a method for analyzing an analyte in areas, such as medical diagnosis, by taking advantage of a reaction between a measurement target substance and a molecule that specifically recognizes the measurement target substance, such as an antigen-antibody reaction, nucleic acid hybridization, an enzymatic reaction, coordinate bonding, and adsorption of particulate entities such as biological cells. However, there is a demand for an analysis method which is higher in sensitivity than such known methods and can be performed in an inexpensive, simple, and quick manner.

As an example of such an analysis method, there has been proposed an analysis method using a composite of an antibody and a structure having particular optical properties. Specifically, a composite which changes in optical properties when an antibody binds to an antigen can be configured by adopting a particular structure as the structure having particular optical properties. The adoption of such a method enables an analysis without performing a complicated operation such as labeling of a sample. Examples of such a structure may include a structure having a photonic crystal structure and a structure having a microscopic structure capable of expressing localized surface plasmon resonance (Patent Literatures 1 and 2).

Citation List

Patent Literature

Patent Literature 1: Japanese Patent Application Laid-Open No. 2011-80848 A

Patent Literature 2: Japanese Patent Application Laid-Open No. 2010-197046 A

US 2003/0017580 A1 describes a method for producing a colorimetric resonant reflection biosensor on rigid surfaces.

US 2006/0180750 A1 describes a single mode fiber optical reader system and method for interrogating a resonant waveguide-grating sensor.

Summary

Technical Problem

However, the methods disclosed in Patent Literatures 1 and 2 still have the problem that the production of a sensor used in the analysis is complicated. Accordingly, there is a demand for an analysis method which has a detection limit and quantitative determination accuracy being equivalent to or higher than those of known methods and which can be performed in an inexpensive and simple manner.

Therefore, an object of the present invention is to provide: an analysis method of an analyte, which has a high detection limit and high quantitative determination accuracy and can be performed in an inexpensive and simple manner; an analysis instrument used for such an analysis method; and a method for producing such an analysis instrument.

Solution to Problem

The present inventor conducted research for solving the aforementioned problem. As a result, the inventor has found that the problem can be solved by adopting a specific analysis plate as an analysis plate for performing such analysis. The present invention has been achieved on the basis of such knowledge.

That is, the present invention is as defined by the appended independent claims. Particular embodiments of the invention are defined in the appended dependent claims.

## Advantageous Effects of Invention

With the analysis plate and an analysis method of the present invention, a method for analyzing an analyte which has a high detection limit and high quantitative determination accuracy and can be performed in an inexpensive and simple manner. By the method for producing an analysis plate of the present invention, such an analysis plate can be easily produced.

## Brief Description of Drawings

FIG. 1 is a cross-sectional view schematically illustrating an example of the analysis plate according to an embodiment of the present invention.
FIG. 2 is a perspective view schematically illustrating the concavo-convex structure 11U of the substrate 111 of the analysis plate illustrated in FIG. 1.
FIG. 3 is a graph illustrating an example of the relationship between the band of the light reflection caused by the selective reflectivity of the selective reflection layer and the band of the light reflection caused by the concavo-convex structure in the analysis plate according to an embodiment of the present invention.
FIG. 4 is a cross-sectional view schematically illustrating another example of the analysis plate according to an embodiment of the present invention.
FIG. 5 is a cross-sectional view schematically illustrating still another example of the analysis plate according to an embodiment of the present invention.
FIG. 6 is a cross-sectional view schematically illustrating still another example of the analysis plate according to an embodiment of the present invention.

## Description of Embodiments

Hereinafter, the present invention will be described in detail with reference to examples and embodiments. However, the present invention is not limited to the following examples and embodiments and may be freely modified for implementation without departing from the scope of the claims.

### <1. Analysis Plate>

The analysis plate according to the present invention includes the features defined in claim 1.
FIG. 1 is a cross-sectional view schematically illustrating an example of the analysis plate according to an embodiment of the present invention. In FIG. 1, an analysis plate 10 includes a substrate 111, and a specific recognition molecule 121 immobilized on a surface 11U of the substrate 111. In the example of FIG. 1, the substrate 111 of the analysis plate 10 has a concavo-convex structure 11U including a bottom 11B and a top 11T, on the upper-side surface in the drawing. Since FIG. 1 and other drawings are schematic illustration, only several unit structures are illustrated as the concavo-convex structure. However, since the concavo-convex structure is a fine structure, an actual product includes far larger number of the concavo-convex unit structures.
The concavo-convex structure capable of exhibiting a structural color is a concavo-convex structure in which the size and repeating period are adjusted such that light within a specific wavelength range is reflected by phenomena such as an optical interference. The structural color is not necessarily limited to a color within the visible range, but may be usually a color within the visible range from the viewpoint of facilitating the production and observation of the analysis plate. Such a concavo-convex structure capable of exhibiting a structural color includes a structure which is called a so-called photonic crystal structure. Specifically, the concavo-convex structure capable of exhibiting a structural color may be a structure in which a unit structure such as a recess is periodically laid out in one or two or more directions in the plane of the substrate surface.
FIG. 2 is a perspective view schematically illustrating the concavo-convex structure 11U of the substrate 111 of the analysis plate illustrated in FIG. 1. The cross-sectional view in FIG. 1 corresponds to a cross section cutting the substrate 111 along a plane passing through a line L1 and vertical to the main surface of the substrate 111 in FIG. 2. In the example of FIG. 2, many rectangular recesses are formed on the surface of the substrate 111 thereby to form the concavo-convex structure 11U including the bottom 11B and the top 11T. The recesses are laid out periodically in two directions that are a direction along the line L1 and a direction along a line L2 orthogonal to the line L1. When a repeating period P1 in the direction along the line L1 and a repeating period P2 in the direction along the line L2 are set to respective appropriate values, the concavo-convex structure can reflect light within a desired wavelength range.
The repeating period of the concavo-convex structure may be appropriately set so that light within a desired

wavelength range is reflected. Specifically, the repeating period of the concavo-convex structure may be a length which is roughly proportional to a desired wavelength of light to be reflected. For example, from the viewpoint of facilitating observation, it is preferable that the band of the light reflection caused by the concavo-convex structure includes the band close to green color, and more specifically includes reflection in a wavelength range of 495 nm to 570 nm. The repeating period of the concavo-convex structure for achieving such reflection may be preferably in a range of 8.090 $\mu$m $\pm$ 0.123 $\mu$m. When two or more repeating periods exist as in the example of FIG. 2, it is preferable that one or more of the repeating periods are within the aforementioned range. However, the repeating period of the concavo-convex structure is not limited to this range. For example, the light reflection band of red color may be obtained as a wider repeating period, or the light reflection band of blue color may be obtained as a narrower repeating period.

When the concavo-convex structure is a structure including a planar surface and many recesses disposed in the plane of the planar surface as in the example of FIG. 2, the shape of the recesses is not particularly limited and may be appropriately selected as long as a desired optical effect can be obtained, and the production is easy. A specific example thereof may include, in addition to the rectangular shape illustrated in FIG. 2, shapes such as a cylindrical recess shape. The depth of the recesses is also not particularly limited and may be appropriately set as long as a desired optical effect can be obtained, and the production is easy. Specifically, the depth of the recesses may be preferably 0.03 $\mu$m to 10 $\mu$m, and more preferably 0.1 $\mu$m to 9 $\mu$m.

As the specific recognition molecule that the analysis plate according to the present invention has, there may be adopted a molecule capable of specifically binding to a measurement target substance in an analyte. Examples of the specific recognition molecule may include proteins such as enzymes and antibodies, nucleic acids such as DNA and RNA, sugar chains, peptide nucleic acids, molecules capable of forming a coordinate bond such as a chelate, and composites thereof. A typical example of the specific recognition molecule may be an antibody capable of undergoing an antigen-antibody reaction with a measurement target substance as an antigen. As another example, when the measurement target substance is a gene, the specific recognition molecule may be a nucleic acid capable of hybridizing with the gene. If necessary, the specific recognition molecule may be chemically modified so that it is immobilized onto the surface of the substrate.

In a preferable aspect of the present invention, the specific recognition molecule is immobilized on the surface having a concavo-convex structure of the substrate. Accordingly, the surface of the analysis plate thus has a microscopic structure defined by the concavo-convex structure and the specific recognition molecule. When a measurement target substance binds to the specific recognition molecule of the analysis plate, the microscopic structure on the surface of the analysis plate changes. Consequently, the optical properties on the surface of the analysis plate particularly acutely changes. Therefore, with such a structure, a measurement target substance can be qualitatively and/or quantitatively measured in a simple and quick manner.

In the analysis plate according to the present invention, the substrate includes a selective reflection layer in at least part of the layer thereof. The selective reflection layer is a layer which reflects only light within a specific wavelength range and transmits light having other wavelengths, of light having entered the layer, due to the molecular structure in the layer. In the example of FIG. 1, the entirety of the substrate 111 is constituted by the selective reflection layer.

As the selective reflection layer, a linearly polarized light separation layer or a circularly polarized light separation layer may be used. The linearly polarized light separation layer is a layer capable of reflecting a certain linearly polarized light component of non-polarized light and transmitting other components. The circularly polarized light separation layer is a layer having a circularly polarized light separation function, that is, a layer capable of reflecting a certain circularly polarized light component of non-polarized light and transmitting other components. An example of the linearly polarized light separation layer may be a layer having a structure in which a large number of layers of a plurality of types each having a different refractive index are stacked on one another. An example of the circularly polarized light separation layer may be a resin layer having cholesteric regularity (hereinafter, sometimes simply referred to as a "cholesteric resin layer"). Since the reflective wavelength region and reflective wavelength bandwidth of such a linearly polarized light separation layer or circularly polarized light separation layer are easily adjusted to desired ones during the production process, these layers are advantageously used as the selective reflection layer in the present invention. Of these, the cholesteric resin layer is particularly excellent in that the selective reflection layer having desired reflective wavelength region and reflective wavelength bandwidth can be easily formed.

When the analysis plate according to the present invention is used, binding of the measurement target substance to the specific recognition molecule causes the optical properties on the surface of the analysis plate to change. Such a change in optical properties can be clearly observed when the substrate includes the selective reflection layer. As a result, the detection limit and quantitative determination accuracy of the analysis can become higher than those in which the selective reflection layer is not included.

According to the present invention, the surface of the substrate has the concavo-convex structure capable of exhibiting a structural color, and the band of the light reflection caused by the selective reflectivity of the selective reflection layer falls within the band of the light reflection caused by the concavo-convex structure. The peak of the band of the light reflection caused by the selective reflectivity falls within the band of the light reflection caused by the concavo-convex structure. Preferably, the peak of the band of the light reflection caused by the selective reflectivity falls within the band of the half width of the light reflection caused by the concavo-convex structure. When the selective reflection layer has such a reflection band, the detection limit and quantitative determination accuracy of the analysis can become still higher than those when the selective reflection layer is not included.

FIG. 3 is a graph illustrating an example of the relationship between the band of the light reflection caused by the selective reflectivity of the selective reflection layer and the band of the light reflection caused by the concavo-convex structure in the analysis plate according to an embodiment of the present invention. In the example of FIG. 3, the profile of the light reflection caused by the concavo-convex structure of the analysis plate is indicated by a line 311, and the profile of the light reflection caused by the selective reflectivity is indicated by a line 321. In the example of FIG. 3, both profile 311 for the light reflection caused by the concavo-convex structure and profile 321 for the light reflection caused by the selective reflectivity have their peaks at a wavelength $\lambda$. Furthermore, in the example of FIG. 3, the profile for the light reflection caused by the concavo-convex structure in a state where a measurement target substance binds to the specific recognition molecule of the analysis plate is indicated by a broken line 312.

As previously described, when a measurement target substance binds to the specific recognition molecule of the analysis plate, the microscopic structure on the surface of the analysis plate changes in size and/or periodicity. Accordingly, the optical properties based on the concavo-convex structure changes. For example, as indicated by the broken line 312 in FIG. 3, the reflection intensity decreases. Therefore, the binding of the measurement target substance to the specific recognition molecule can be detected by measuring the light reflection amount at the wavelength $\lambda$. When the analysis plate includes, as the selective reflection layer, a layer having the light reflection profile indicated by the line 321, a change of the reflection intensity in the wavelength region around the wavelength $\lambda$ can be more clearly observed. In addition, the degree to which observation of the vicinity of the wavelength $\lambda$ is disturbed by influence of the reflection in a wavelength region far from the wavelength $\lambda$ can be reduced. As a result, the detection limit and quantitative determination accuracy of the analysis can become higher than those when the selective reflection layer is not included.

When the selective reflection layer is positioned on the surface having a concavo-convex structure and therefore the selective reflection layer constitutes the concavo-convex structure as in the example illustrated in FIG. 1, the profile of the light reflection when the analysis plate is observed is usually observed as a sum of the profile of the light reflection caused by the concavo-convex structure and the profile of the light reflection caused by the selective reflectivity of the selective reflection layer. Therefore, it is difficult to separately measure these profiles as shown in the graph of FIG. 3. However, even in such a case, the profile of the light reflection caused by the selective reflectivity of the selective reflection layer of the analysis plate may be measured by forming a flat selective reflection layer without the concavo-convex structure formed of the same material as the selective reflection layer of the analysis plate, and measuring the profile of the light reflection of this flat selective reflection layer. Similarly, the profile of the light reflection caused by the concavo-convex structure in such a case may be measured by forming a substrate which has the same concavo-convex structure as the concavo-convex structure of the analysis plate and which is formed of another material not having selective reflectivity, and measuring the profile of the light reflection of this substrate. Alternatively, these profiles may also be obtained by known information or simulation.

From the viewpoint of more clearly observing a change in reflection intensity, the bandwidth of the light reflection caused by selective reflectivity is preferably narrow to a certain extent or more. Specifically, a half width $W_1$ of the band of the light reflection caused by the concavo-convex structure and a half width $W_2$ of the band of the light reflection caused by selective reflectivity preferably have a relationship of $W_2 < (W_1 \times 1.5)$. The lower limit value for the $W_2$ is not particularly limited, but may be, for example, $(W_1 \times 0.2) < W_2$. The half widths $W_1$ and $W_2$ are each the width of the reflection band in which the reflection intensity is 1/2 or more the peak reflection intensity at its peak wavelength. Specifically explaining referring to the example of FIG. 3, the half width $W_1$ of the band of the light reflection indicated by the line 311 corresponds to the width of the peak at a midpoint M31 between a base line R0 and a peak height R31 of 311, and the half width $W_2$ of the band of the light reflection indicated by the line 321 corresponds to the width of the peak at a midpoint M32 between the base line R0 and a peak height R32 of 321.

<2. Variation Of Analysis Plate>

Although the substrate 111 of the analysis plate illustrated in FIG. 1 includes only the selective reflection layer, the substrate in the analysis plate according to the present invention is not limited to this, and may have another configuration, within the scope of the appended claims.

For example, the substrate may include, as illustrated in an analysis plate 40 of FIG. 4, a selective reflection layer and a support layer which supports the selective reflection layer. The analysis plate 40 illustrated in FIG. 4 is different from the analysis plate 10 illustrated in FIG. 1 in an aspect of having as the substrate a substrate 410 having a multi-layer structure, which is different from that of the substrate 111, and has the same structure in other aspects. The substrate 410 of the analysis plate 40 includes a selective reflection layer 411 having a concavo-convex structure and a support layer 412 which supports the selective reflection layer 411. As the support layer 412, there may be used

a film-shaped or plate-shaped structure which is transparent and has a strength higher than the selective reflection layer 411. When such a support layer 412 is included, there may be obtained an analysis plate which has high mechanical strength while having the aforementioned advantage of the present invention.

In the analysis plates illustrated in FIG. 1 and FIG. 4, the selective reflection layer is positioned on the surface having a concavo-convex structure. Accordingly, the selective reflection layer constitutes the concavo-convex structure. With such a configuration, the binding of a measurement target substance to the specific recognition molecule can be clearly detected. However, the analysis plate according to the present invention is not limited to this, and, within the scope of the appended claims, a layer other than the selective reflection layer may be positioned on the surface of the substrate to constitute the concavo-convex structure.

For example, as illustrated in an analysis plate 50 of FIG. 5, the substrate may include: a layer having a concavo-convex structure other than a selective reflection layer; and a flat selective reflection layer. The analysis plate 50 of FIG. 5 is different from the analysis plate 10 illustrated in FIG. 1 in an aspect of having a substrate 510 having a multi-layer structure, which is different from the substrate 111, and has the same structure in other aspects. The substrate 510 of the analysis plate 50 includes: a transparent layer 513 having a concavo-convex structure; and a selective reflection layer 511 disposed on the surface of the layer 513 opposite to the surface having a concavo-convex structure. Also with such a configuration, the detection limit and quantitative determination accuracy of the analysis can become higher than those when the selective reflection layer is not included.

In the aforementioned examples, explanation has been made with the analysis plate having a flat surface at macroscopical level, although the plate has a concavo-convex structure at nano-level capable of exhibiting a structural color. However, the analysis plate according to the present invention is not limited to this, and, within the scope of the appended claims, can further have an optional configuration which is suitable for analysis, as necessary. For example, the analysis plate may have a well for receiving a liquid analyte, such as an analysis plate 60 illustrated in FIG. 6.

The analysis plate 60 illustrated in FIG. 6 includes: a structure that is the same as that of the analysis plate 40 illustrated in FIG. 4; and a plate-shaped structure 631 disposed on the surface having a concavo-convex structure thereof. Therefore, the analysis plate 60 includes: the substrate 410 having the selective reflection layer 411 having the concavo-convex structure 11U and the support layer 412 supporting the selective reflection layer 411; and a specific recognition molecule (not illustrated) immobilized on the surface having a concavo-convex structure of the substrate 410.

The plate-shaped structure 631 disposed on the surface of the substrate 410 is a flat plate-shaped structure having many through holes 63H. The plate-shaped structure 631 may be disposed on the surface of the substrate 410 by bonding a bottom 63D of the plate-shaped structure 631 to the surface of the substrate 410 via an appropriate adhesive layer (not illustrated) as necessary. With such a structure, there are formed wells defined by the through holes 63H and the surface having the concavo-convex structure 11U. By pouring the liquid analytes into the wells, efficient analysis of many analytes can be performed.

In addition to the aforementioned constituents, within the scope of the appended claims, the analysis plate according to the present invention may further include an optional constituent for further facilitating an analysis operation.

An example of the optional constituent may be an electrode for detecting existence of an analyte, a measurement target substance, or a component other than a measurement target substance in an analyte using an electric device. For example, when blood is an analyte, it is possible to perform an analysis wherein a certain component in blood is analyzed as a measurement target substance through a reaction with the specific recognition molecule, while another component is analyzed using such an electrode.

Another example of the optional constituent may be a tag such as an RFID tag for managing data of a large number of analytes.

<3. Analysis Method>

The analysis method according to the present invention is an analysis method for measuring a measurement target substance contained in an analyte, and includes the following steps.

Step (A): preparing an analysis plate according to the present invention which includes as the specific recognition molecule a molecule that specifically binds to a measurement target substance.

Step (B): bringing an analyte into contact with the analysis plate.

Step (C): measuring a change in light reflection amount of the surface of the analysis plate caused by the contact with the analyte.

The step (A) may be performed by appropriately preparing a desired specific recognition molecule by a known method and producing an analysis plate with the prepared specific recognition molecule. The specific method for producing the analysis plate will be described in detail later.

In the step (B), the analyte may be any analyte required to be analyzed on whether a measurement target substance is contained. The analyte is usually in a liquid state. The analyte may be various samples as they are, or may be samples having been subjected to a treatment such as adding a solvent, purification, pH adjustment, an anticoagulation

treatment, and pulverization for a solid sample, as necessary. Examples of the sample may include blood, urine, feces, spinal fluid, sputum, organs, cell homogenation liquid, saliva, sweat, skin, and exudate sampled from living bodies such as human bodies, as well as microorganisms such as bacteria, viruses, and yeast, and cultures of other cells. Further examples of the sample may include a sample that is required to be quantitatively and/or qualitatively analyzed as to whether it contains a measurement target substance, such as an object sampled from the environment and a reaction mixture obtained by a chemical reaction. By subjecting such analytes to the analysis, useful analysis of an analyte in various fields such as analysis in a field of medical diagnosis and other fields can be performed. Examples of the measurement target substance that may be contained in an analyte may include chemical substances such as various proteins, sugar chains, nucleic acids, and metal atoms, as well as particulate objects such as microorganisms such as bacteria, viruses, and yeast, and other cells.

The step (B) is performed such that an analyte is brought into contact with the surface of the analysis plate to which the specific recognition molecule is immobilized. When a measurement target substance is contained in an analyte, the measurement target substance and the specific recognition molecule bind to each other in the step (B). Accordingly, the optical properties of the surface of the analysis plate change. In particular, when the surface of the substrate has the concavo-convex structure capable of exhibiting a structural color, the microscopic structure of the surface of the analysis plate is changed by the binding. Accordingly, the optical properties of the surface of the analysis plate can particularly acutely change.

The specific operation of the step (B) is not particularly limited, and may be performed according to any optional operation. For example, the operation may be performed simply by dropping an analyte onto the surface of the analysis plate. Alternatively, for example, an analyte may be placed on the analysis plate and maintained as it is, thereby to track a time-dependent change of the amount of a measurement target substance in the analyte. For example, an analyte containing a specific cell as a measurement target substance may be used to continuously perform the analysis method according to the present invention while culturing the cell, thereby to track a time-dependent change of the cell amount due to the culturing.

The step (C) may be performed by measuring the color tone on the analysis plate using a known device such as a spectrometer. Examples of the spectrometer to be used may include a fiber-type spectrophotometer, a UV-visible spectrophotometer, and a color meter. The step (C) may also be performed using a calculating device provided with a general-purpose camera for acquiring images and a program for analyzing the image data acquired by the camera. More specifically, the step (C) may also be performed using a general-purpose device installed with a program for analyzing image data, such as a smart phone equipped with a camera. Alternatively, when high quantitative accuracy is not required, the step (C) may also be performed simply by visually observing a change in color of the analysis plate. From the aforementioned reason, the use of the analysis plate according to the present invention enables clear observation of a change in reflection amount caused by the contact with an analyte, and allows for analysis with a detection limit and quantitative determination accuracy being higher than a case where the selective reflection layer is not included.

<4. Method For Producing Analysis Plate, And Material And Others Of Each Constituent>

The analysis plate according to the present invention may be produced by any production method falling within the scope of appended claim 7. Specifically, the analysis plate according to the present invention may be produced by a production method according to claim 7 including a step of producing the aforementioned specific substrate and a step of immobilizing a specific recognition molecule on the surface of the substrate.

<4.1. Substrate>

As the material of the substrate, a known material may be appropriately selected for use. When the substrate includes a layer having a concavo-convex structure, a known material suitable for forming such a concavo-convex structure may be appropriately selected for use as the material of the layer having a concavo-convex structure. From the viewpoint of forming a microscopic concavo-convex structure and maintaining the structure after the formation thereof, a resin other than a thermoplastic resin is preferable rather than the thermoplastic resin. For example, a thermosetting resin and an energy ray curable resin such as a UV curable resin are preferable.

When the substrate includes a layer other than the layer having a concavo-convex structure (for example, the support layer 412 in the example illustrated in FIG. 4), the specific material of such a layer may be appropriately selected for use from known resins and the like. This resin contains a polymer and, as necessary, an optional component. Examples of the polymer contained in the resin may include a chain olefin polymer, a cycloolefin polymer, polycarbonate, polyester, polysulfone, polyether sulfone, polystyrene, polyvinyl alcohol, a cellulose acetate-based polymer, polyvinyl chloride, polymethacrylate, and polyethylene glycol. As the aforementioned polymer, one type thereof may be solely used, and two or more types thereof may also be used in combination at any ratio. When the analysis plate according to the present invention includes a constituent other than the substrate and the specific recognition molecule (for example,

the plate-shaped structure 631 illustrated in FIG. 6), the material of such a constituent may also be appropriately selected for use from known resins and the like such as those having been described as examples.

Examples of a processing method for forming the concavo-convex structure may include a processing method of transferring a pattern having a concavo-convex structure onto the substrate or the material of the substrate and a processing method of using a fine processing device such as a laser. An example of the method for forming a concavo-convex structure may be a method of forming an original plate constituted of the selective reflection layer or a plurality of layers containing the selective reflection layer and then forming a concavo-convex structure on the surface of the original plate. Another example of the method for producing the substrate may be a method of semi-curing the material of the substrate to obtain a semi-cured layer, forming a concavo-convex structure capable of exhibiting a structural color on the surface of the semi-cured layer, and further fully curing the semi-cured layer. When a cholesteric resin layer is adopted as the material of the selective reflection layer, such a method including the combination of semi-curing and full-curing may be particularly preferably adopted. A polymerizable liquid crystal compound capable of exhibiting a cholesteric liquid crystal phase, and a method for producing the substrate containing the selective reflection layer with the polymerizable liquid crystal compound will be described in detail later.

<4.2. Immobilization Of Specific Recognition Molecule>

The specific recognition molecule may be appropriately produced by a known biochemical method. The method for immobilizing the specific recognition molecule on the surface of the substrate is not particularly limited, and a known method may be appropriately adopted. For example, when an antibody is used as the specific recognition molecule, immobilization of the antibody onto the surface of the substrate may be performed by physical adsorption and/or chemical adsorption, and the like.

<4.3. Cholesteric Resin Layer, Material Thereof, And Method For Producing Substrate Containing Selective Reflection Layer Using The Material>

As the selective reflection layer constituting the substrate of the analysis plate according to the present invention, there may be particularly preferably used a cholesteric resin layer obtained by polymerizing a polymerizable liquid crystal compound capable of exhibiting a cholesteric liquid crystal phase. The adoption of the cholesteric resin layer as the selective reflection layer facilitates the formation of the selective reflection layer having desired reflective wavelength region and reflective wavelength bandwidth.

Since the cholesteric resin layer has a circularly polarized light separation function, the formation of the selective reflection layer with the cholesteric resin layer enables production of a selective reflection layer having a circularly polarized light separation function. Since the cholesteric resin layer can have such a circularly polarized light separation function in a certain specific narrow wavelength region, it may be preferably used as the selective reflection layer according to the present invention. This wavelength region in which circularly polarized light can be reflected is referred to as a selective reflection band.

<4.3.1. Polymerizable Liquid Crystal Compound>

As the polymerizable liquid crystal compound capable of exhibiting a cholesteric liquid crystal phase for use, an appropriate compound may be appropriately selected from known polymerizable liquid crystal compounds. A particularly preferable example of the polymerizable liquid crystal compound may include the polymerizable liquid crystal compound represented by the following formula (I). The polymerizable liquid crystal compound represented by the formula (I) is sometimes appropriately referred to as a "compound (I)" below. Since the compound (I) exhibits liquid crystal properties, the compound (I) can develop a liquid crystal phase when oriented. In addition, since this compound (I) is polymerizable, it may be polymerized in a state of exhibiting the liquid crystal phase, thereby becoming a polymer while maintaining the molecular orientation in the liquid crystal phase. Therefore, the cholesteric resin layer having a circularly polarized light separation function can be obtained by polymerizing the compound (I) in a state in which the compound (I) exhibits the cholesteric phase.

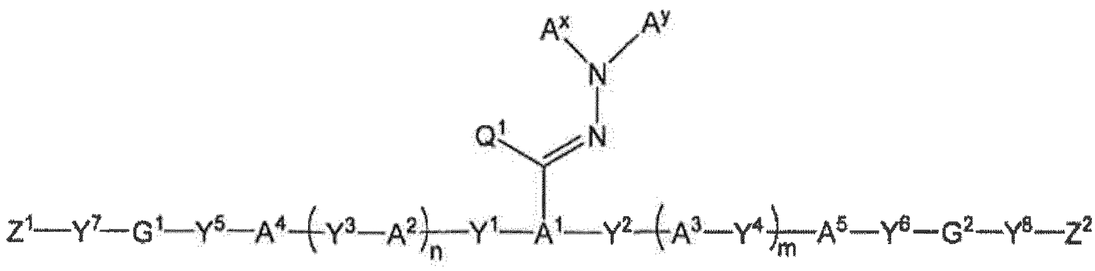

(I)

Usually in the compound (I), a group $-Y^5-A^4-(Y^3-A^2)_n-Y^1-A^1-Y^2-(A^3-Y^4)_m-A^5-Y^6-$ acts as the main chain mesogen, and a group $>A^1-C(Q^1)=N-N(A^x)A^y$ acts as the side chain mesogen. The group $A^1$ affects natures of both the main chain mesogen and the side chain mesogen.

In the formula (I) mentioned above, $Y^1$ to $Y^8$ are each independently a chemical single bond, -O-, -S-, -O-C(=O)-, -C(=O)-O-, -O-C(=O)-O-, $-NR^1-C(=O)-$, $-C(=O)-NR^1-$, $-O-C(=O)-NR^1-$, $-NR^1-C(=O)-O-$, $-NR^1-C(=O)-NR^1-$, $-O-NR^1-$, or $-NR^1-O-$.

Herein, $R^1$ is a hydrogen atom or an alkyl group of 1 to 6 carbon atoms.

Examples of the alkyl group of 1 to 6 carbon atoms of $R^1$ may include a methyl group, an ethyl group, a n-propyl group, an isopropyl group, a n-butyl group, a sec-butyl group, a t-butyl group, a n-pentyl group, and a n-hexyl group.

It is preferable that $R^1$ is a hydrogen atom or an alkyl group of 1 to 4 carbon atoms.

In the compound (I), it is preferable that $Y^1$ to $Y^8$ are each independently a chemical single bond, -O-, -O-C(=O)-, -C(=O)-O-, or -O-C(=O)-O-.

In the formula (I) mentioned above, $G^1$ and $G^2$ are each independently a divalent aliphatic group of 1 to 20 carbon atoms optionally having a substituent.

Examples of the divalent aliphatic group of 1 to 20 carbon atoms may include a divalent aliphatic group having a linear structure, such as an alkylene group of 1 to 20 carbon atoms and an alkenylene group of 2 to 20 carbon atoms; and a divalent aliphatic group, such as a cycloalkanediyl group of 3 to 20 carbon atoms, a cycloalkenediyl group of 4 to 20 carbon atoms, and a divalent alicyclic fused ring group of 10 to 30 carbon atoms.

Examples of the substituent in the divalent aliphatic group of $G^1$ and $G^2$ may include a halogen atom, such as a fluorine atom, a chlorine atom, a bromine atom, and an iodine atom; and an alkoxy group of 1 to 6 carbon atoms, such as a methoxy group, an ethoxy group, a n-propoxy group, an isopropoxy group, a n-butoxy group, a sec-butoxy group, a t-butoxy group, a n-pentyloxy group, and a n-hexyloxy group. Among these, a fluorine atom, a methoxy group, and an ethoxy group are preferable.

The aforementioned aliphatic groups may have one or more per one aliphatic group of -O-, -S-, -O-C(=O)-, -C(=O)-O-, -O-C(=O)-O-, $-NR^2-C(=O)-$, $-C(=O)-NR^2-$, $-NR^2-$, or -C(=O)- inserted therein. However, cases where two or more -O- or -S- are adjacently inserted are excluded. Herein, $R^2$ is a hydrogen atom or an alkyl group of 1 to 6 carbon atoms that are the same as those for the aforementioned $R^1$. It is preferable that $R^2$ is a hydrogen atom or a methyl group.

It is preferable that the group inserted into the aliphatic groups is -O-, -O-C(=O)-, -C(=O)-O-, or -C(=O)-.

Specific examples of the aliphatic groups into which the group is inserted may include $-CH_2-CH_2-O-CH_2-CH_2-$, $-CH_2-CH_2-S-CH_2-CH_2-$, $-CH_2-CH_2-O-C(=O)-CH_2-CH_2-$, $-CH_2-CH_2-C(=O)-O-CH_2-CH_2-$, $-CH_2-CH_2-C(=O)-O-CH_2-$, $-CH_2-O-C(=O)-O-CH_2-CH_2-$, $-CH_2-CH_2-NR^2-C(=O)-CH_2-CH_2-$, $-CH_2-CH_2-C(=O)-NR^2-CH_2-$, $-CH_2-NR^2-CH_2-CH_2-$, and $-CH_2-C(=O)-CH_2-$.

Among these, from the viewpoint of more favorably expressing the desired effect of the present invention, $G^1$ and $G^2$ are each independently preferably a divalent aliphatic group having a linear structure, such as an alkylene group of 1 to 20 carbon atoms and an alkenylene group of 2 to 20 carbon atoms, more preferably an alkylene group of 1 to 12 carbon atoms, such as a methylene group, an ethylene group, a trimethylene group, a propylene group, a tetramethylene group, a pentamethylene group, a hexamethylene group, an octamethylene group, and a decamethylene group $[-(CH_2)_{10}-]$, and particularly preferably a tetramethylene group $[-(CH_2)_4-]$, a hexamethylene group $[-(CH_2)_6-]$, an octamethylene group $[-(CH_2)_8-]$, or a decamethylene group $[-(CH_2)_{10}-]$.

In the formula (I) mentioned above, $Z^1$ and $Z^2$ are each independently an alkenyl group of 2 to 10 carbon atoms that may be unsubstituted or substituted by a halogen atom.

It is preferable that the number of carbon atoms in the alkenyl group is 2 to 6. Examples of the halogen atom that is a substituent in the alkenyl group of $Z^1$ and $Z^2$ may include a fluorine atom, a chlorine atom, and a bromine atom. A chlorine atom is preferable.

Specific examples of the alkenyl group of 2 to 10 carbon atoms of $Z^1$ and $Z^2$ may include $CH_2=CH-$, $CH_2=C(CH_3)-$, $CH_2=CH-CH_2-$, $CH_3-CH=CH-$, $CH_2=CH-CH_2-CH_2-$, $CH_2=C(CH_3)-CH_2-CH_2-$, $(CH_3)_2C=CH-CH_2-$, $(CH_3)_2C=CH-$

$CH_2$-$CH_2$-, $CH_2$=C(Cl)-, $CH_2$=C($CH_3$)-$CH_2$-, and $CH_3$-CH=CH-$CH_2$-.

Among these, from the viewpoint of favorably expressing the desired effect of the present invention, $Z^1$ and $Z^2$ are each independently preferably $CH_2$=CH-, $CH_2$=C($CH_3$)-, $CH_2$=C(Cl)-, $CH_2$=CH-$CH_2$-, $CH_2$=C($CH_3$)-$CH_2$-, or $CH_2$=C($CH_3$)-$CH_2$-$CH_2$-, more preferably $CH_2$=CH-, $CH_2$=C($CH_3$)- or $CH_2$=C(Cl)-, and particularly preferably $CH_2$=CH-.

In the formula (I) mentioned above, $A^X$ is an' organic group of 2 to 30 carbon atoms having at least one aromatic ring selected from the group consisting of an aromatic hydrocarbon ring and an aromatic heterocyclic ring. The "aromatic ring" means a cyclic structure having aromaticity in the broad sense based on Huckel rule, that is, a cyclic conjugated structure having (4n+2) $\pi$ electrons, and a cyclic structure that exhibits aromaticity by involving a lone pair of electrons of a heteroatom, such as sulfur, oxygen, and nitrogen, in a $\pi$ electron system, typified by thiophene, furan, and benzothiazole.

The organic group of 2 to 30 carbon atoms having at least one aromatic ring selected from the group consisting of an aromatic hydrocarbon ring and an aromatic heterocyclic ring, of $A^x$, may have a plurality of aromatic rings, or have an aromatic hydrocarbon ring and an aromatic heterocyclic ring.

Examples of the aromatic hydrocarbon ring may include a benzene ring, a naphthalene ring, and an anthracene ring. Examples of the aromatic heterocyclic ring may include a monocyclic aromatic heterocyclic ring, such as a pyrrole ring, a furan ring, a thiophene ring, a pyridine ring, a pyridazine ring, a pyrimidine ring, a pyrazine ring, a pyrazole ring, an imidazole ring, an oxazole ring, and a thiazole ring; and a fused aromatic heterocyclic ring, such as a benzothiazole ring, a benzoxazole ring, a quinoline ring, a phthalazine ring, a benzimidazole ring, a benzopyrazole ring, a benzofuran ring, a benzothiophene ring, a thiazolopyridine ring, an oxazolopyridine ring, a thiazolopyrazine ring, an oxazolopyrazine ring, a thiazolopyridazine ring, an oxazolopyridazine ring, a thiazolopyrimidine ring, and an oxazolopyrimidine ring.

The aromatic ring of $A^x$ may have a substituent. Examples of the substituent may include a halogen atom, such as a fluorine atom and a chlorine atom; a cyano group; an alkyl group of 1 to 6 carbon atoms, such as a methyl group, an ethyl group, and a propyl group; an alkenyl group of 2 to 6 carbon atoms, such as a vinyl group and an allyl group; a halogenated alkyl group of 1 to 6 carbon atoms, such as a trifluoromethyl group; a substituted amino group, such as a dimethylamino group; an alkoxy group of 1 to 6 carbon atoms, such as a methoxy group, an ethoxy group, and an isopropoxy group; a nitro group; an aryl group, such as a phenyl group and a naphthyl group; -C(=O)-$R^5$; -C(=O)-O$R^5$; and -$SO_2R^6$. Herein, $R^5$ is an alkyl group of 1 to 20 carbon atoms, an alkenyl group of 2 to 20 carbon atoms, or a cycloalkyl group of 3 to 12 carbon atoms. $R^6$ is an alkyl group of 1 to 20 carbon atoms, an alkenyl group of 2 to 20 carbon atoms, a phenyl group, or a 4-methylphenyl group, which are the same as those for $R^4$ which will be described later.

The aromatic ring of $A^x$ may have a plurality of substituents that may be the same or different, and two adjacent substituents may be bonded together to form a ring. The formed ring may be a monocycle or a fused polycycle, and may be an unsaturated ring or a saturated ring.

The "number of carbon atoms" in the organic group of 2 to 30 carbon atoms of $A^x$ means the total number of carbon atoms in the entire organic group which excludes carbon atoms in the substituents (the same applies to $A^y$ which will be described later).

Examples of the organic group of 2 to 30 carbon atoms having at least one aromatic ring selected from the group consisting of an aromatic hydrocarbon ring and an aromatic heterocyclic ring, of $A^x$, may include an aromatic hydrocarbon ring group; an aromatic heterocyclic group; an alkyl group of 3 to 30 carbon atoms having at least one aromatic ring selected from the group consisting of an aromatic hydrocarbon ring group and an aromatic heterocyclic ring group; an alkenyl group of 4 to 30 carbon atoms having at least one aromatic ring selected from the group consisting of an aromatic hydrocarbon ring group and an aromatic heterocyclic ring group; and an alkynyl group of 4 to 30 carbon atoms having at least one aromatic ring selected from the group consisting of an aromatic hydrocarbon ring group and an aromatic heterocyclic ring group.

Preferable specific examples of $A^x$ are as follows. However, $A^x$ is not limited to the following examples. In the following formulae, "-" represents an atomic bonding at any position of the ring (the same applies to the following).

(1) An aromatic hydrocarbon ring group

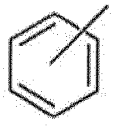
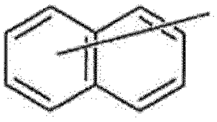
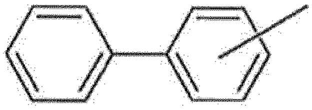

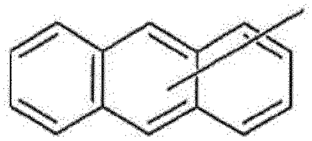
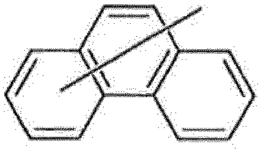
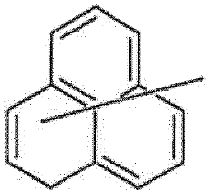

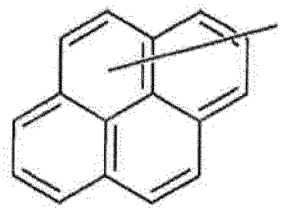
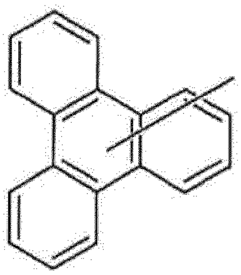
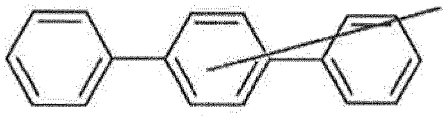

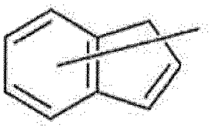
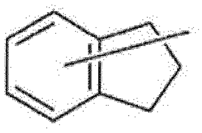
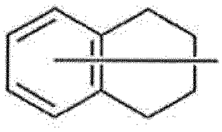

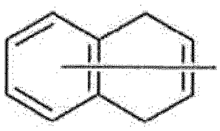
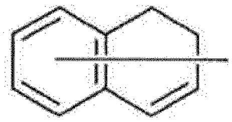
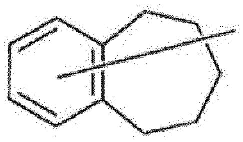

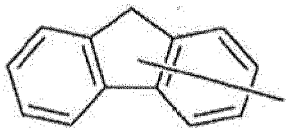
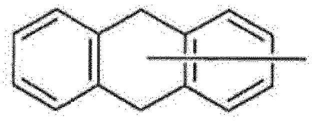

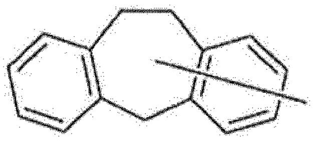
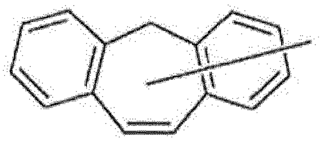

(2) An aromatic heterocyclic group

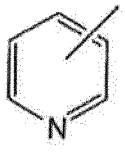 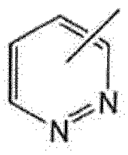 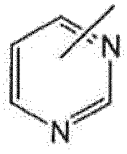 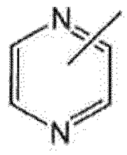

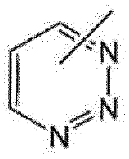 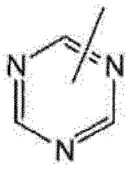 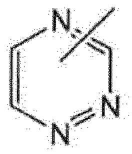

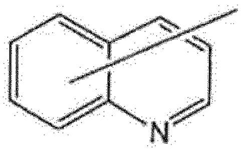 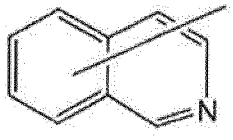 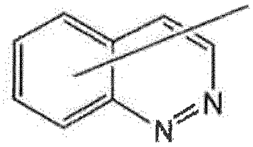

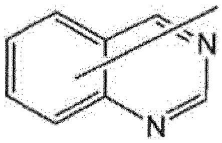 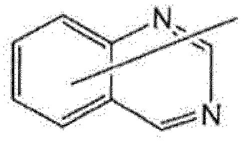 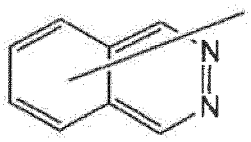

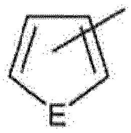 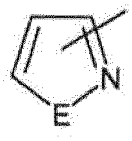 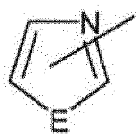

In the aforementioned formulae, E is $NR^{6a}$, an oxygen atom, or a sulfur atom. Herein, $R^{6a}$ is a hydrogen atom; or an alkyl group of 1 to 6 carbon atoms, such as a methyl group, an ethyl group, and a propyl group.

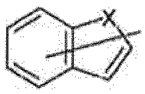 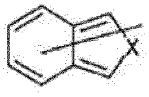 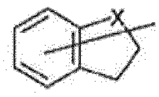 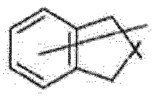 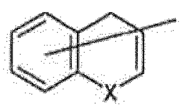 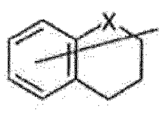

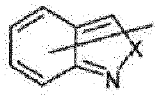 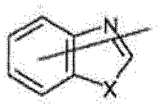 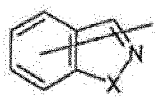 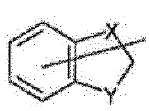 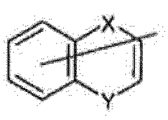

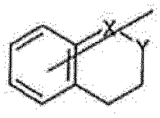 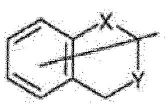 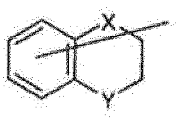 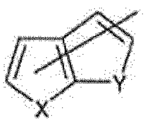 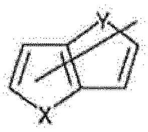

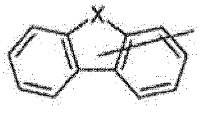 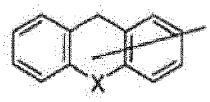 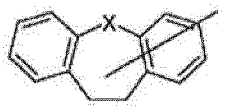 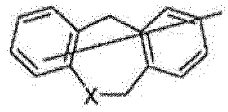

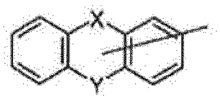 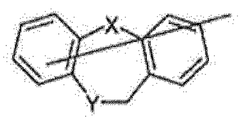 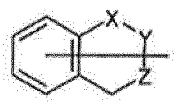 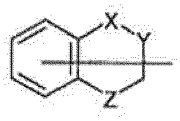

In the aforementioned formulae, X, Y, and Z are each independently $NR^7$, an oxygen atom, a sulfur atom, -SO-, or $-SO_2-$ (provided that cases where an oxygen atom, a sulfur atom, -SO-, and $-SO_2-$ are each adjacent are excluded). $R^7$ is a hydrogen atom, or an alkyl group of 1 to 6 carbon atoms, such as a methyl group, an ethyl group, and a propyl group, which are the same as those for $R^{6a}$ described above.

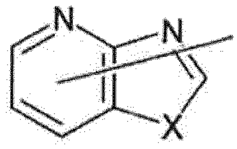
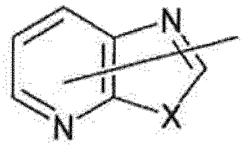
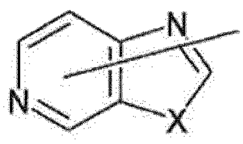
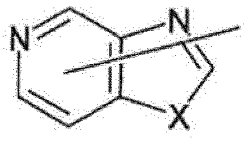
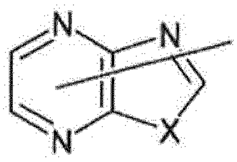
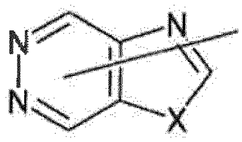
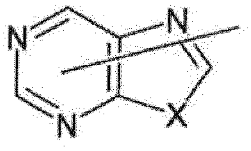
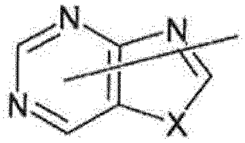

(In the aforementioned formulae, X has the same meanings as described above.)

(3) An alkyl group having at least one aromatic ring selected from the group consisting of an aromatic hydrocarbon ring group and an aromatic heterocyclic ring group

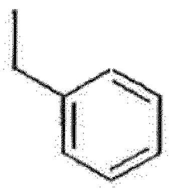
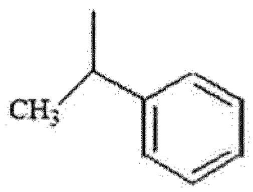
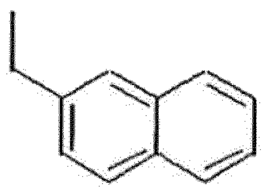
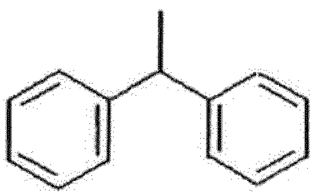
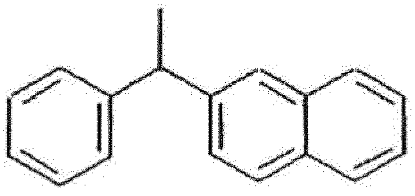
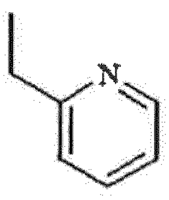
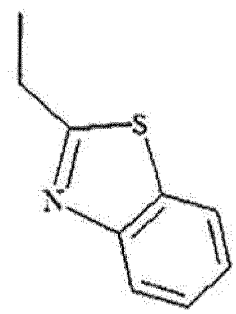
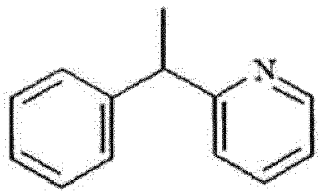

(4) An alkenyl group having at least one aromatic ring selected from the group consisting of an aromatic hydrocarbon ring group and an aromatic heterocyclic ring group

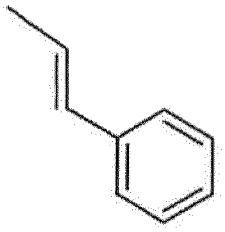
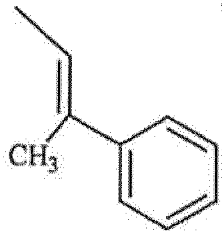
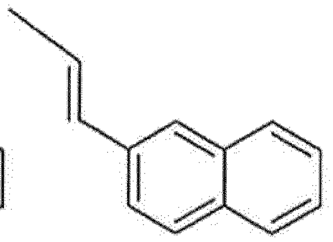
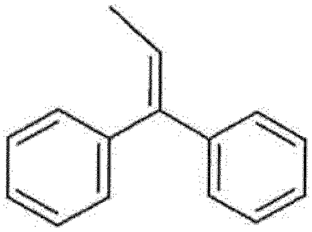
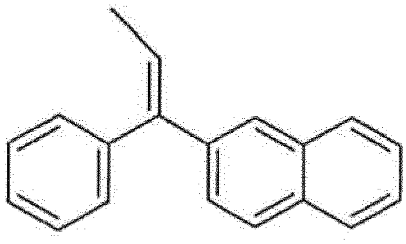

(5) An alkynyl group having at least one aromatic ring selected from the group consisting of an aromatic hydrocarbon ring group and an aromatic heterocyclic ring group

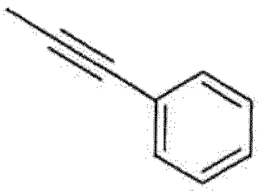
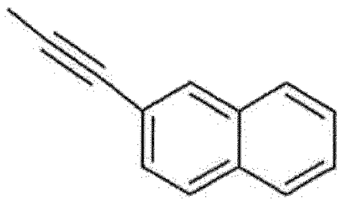

Of $A^x$ described above, an aromatic hydrocarbon group of 6 to 30 carbon atoms and an aromatic heterocyclic group of 4 to 30 carbon atoms are preferable, and any of the groups shown below are more preferable.

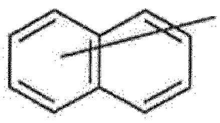
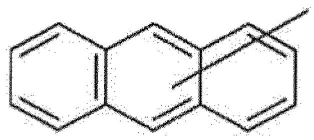

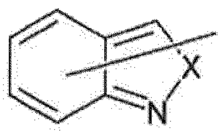 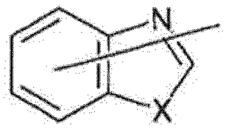 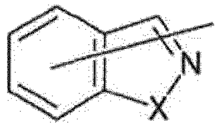 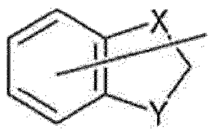

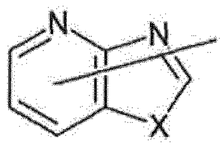 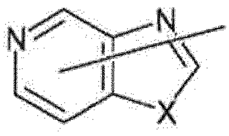 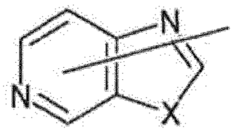 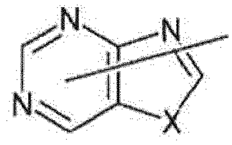

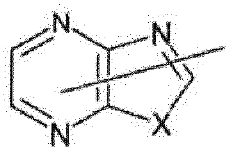 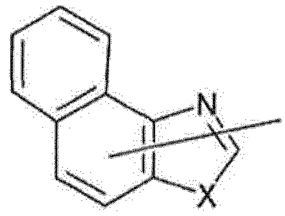 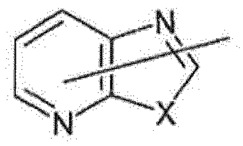

Any of the following groups is further preferable.

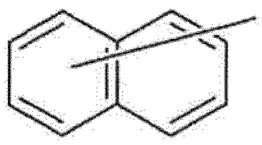 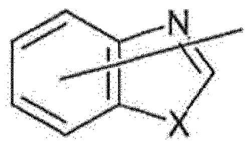

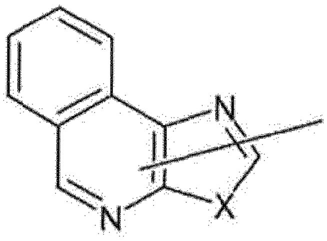 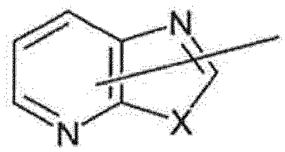 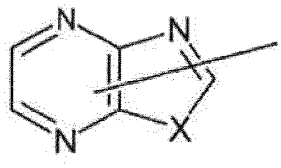

The ring that $A^x$ has may have a substituent. Examples of such a substituent may include a halogen atom, such as a fluorine atom and a chlorine atom; a cyano group; an alkyl group of 1 to 6 carbon atoms, such as a methyl group, an ethyl group, and a propyl group; an alkenyl group of 2 to 6 carbon atoms, such as a vinyl group and an allyl group; a halogenated alkyl group of 1 to 6 carbon atoms, such as a trifluoromethyl group; a substituted amino group, such as a dimethylamino group; an alkoxy group of 1 to 6 carbon atoms, such as a methoxy group, an ethoxy group, and an isopropoxy group; a nitro group; an aryl group, such as a phenyl group and a naphthyl group; $-C(=O)-R^8$; $-C(=O)-OR^8$; and $-SO_2R^6$. Herein, $R^8$ is an alkyl group of 1 to 6 carbon atoms, such as a methyl group and an ethyl group; or an aryl group of 6 to 14 carbon atoms, such as a phenyl group. In particular, it is preferable that the substituent is a halogen atom, a cyano group, an alkyl group of 1 to 6 carbon atoms, or an alkoxy group of 1 to 6 carbon atoms.

The ring that $A^x$ has may have a plurality of substituents that may be the same or different, and two adjacent substituents may be bonded together to form a ring. The formed ring may be a monocycle or a fused polycycle.

The "number of carbon atoms" in the organic group of 2 to 30 carbon atoms of $A^x$ means the total number of carbon atoms in the entire organic group which excludes carbon atoms in the substituents (the same applies to $A^y$ which will be described later).

In the aforementioned formula (I), $A^y$ is a hydrogen atom, an alkyl group of 1 to 20 carbon atoms optionally having a substituent, an alkenyl group of 2 to 20 carbon atoms optionally having a substituent, a cycloalkyl group of 3 to 12 carbon atoms optionally having a substituent, an alkynyl group of 2 to 20 carbon atoms optionally having a substituent, $-C(=O)-R^3$, $-SO_2-R^4$, $-C(=S)NH-R^9$, or an organic group of 2 to 30 carbon atoms having at least one aromatic ring selected from the group consisting of an aromatic hydrocarbon ring and an aromatic heterocyclic ring. Herein, $R^3$ is an alkyl group of 1 to 20 carbon atoms optionally having a substituent, an alkenyl group of 2 to 20 carbon atoms optionally having a substituent, a cycloalkyl group of 3 to 12 carbon atoms optionally having a substituent, or an aromatic

hydrocarbon group of 5 to 12 carbon atoms. $R^4$ is an alkyl group of 1 to 20 carbon atoms, an alkenyl group of 2 to 20 carbon atoms, a phenyl group, or a 4-methylphenyl group. $R^9$ is an alkyl group of 1 to 20 carbon atoms optionally having a substituent, an alkenyl group of 2 to 20 carbon atoms optionally having a substituent, a cycloalkyl group of 3 to 12 carbon atoms optionally having a substituent, or an aromatic group of 5 to 20 carbon atoms optionally having a substituent.

Examples of the alkyl group of 1 to 20 carbon atoms in the alkyl group of 1 to 20 carbon atoms optionally having a substituent, of $A^y$, may include a methyl group, an ethyl group, a n-propyl group, an isopropyl group, a n-butyl group, an isobutyl group, a 1-methylpentyl group, a 1-ethylpentyl group, a sec-butyl group, a t-butyl group, a n-pentyl group, an isopentyl group, a neopentyl group, a n-hexyl group, an isohexyl group, a n-heptyl group, a n-octyl group, a n-nonyl group, a n-decyl group, a n-undecyl group, a n-dodecyl group, a n-tridecyl group, a n-tetradecyl group, a n-pentadecyl group, a n-hexadecyl group, a n-heptadecyl group, a n-octadecyl group, a n-nonadecyl group, and a n-icosyl group. The number of carbon atoms in the alkyl group of 1 to 20 carbon atoms optionally having a substituent is preferably 1 to 12, and further preferably 4 to 10.

Examples of the alkenyl group of 2 to 20 carbon atoms in the alkenyl group of 2 to 20 carbon atoms optionally having a substituent, of $A^y$, may include a vinyl group, a propenyl group, an isopropenyl group, a butenyl group, an isobutenyl group, a pentenyl group, a hexenyl group, a heptenyl group, an octenyl group, a decenyl group, an undecenyl group, a dodecenyl group, a tridecenyl group, a tetradecenyl group, a pentadecenyl group, a hexadecenyl group, a heptadecenyl group, an octadecenyl group, a nonadecenyl group, and an icocenyl group. The number of carbon atoms in the alkenyl group of 2 to 20 carbon atoms optionally having a substituent is preferably 2 to 12.

Examples of the cycloalkyl group of 3 to 12 carbon atoms in the cycloalkyl group of 3 to 12 carbon atoms optionally having a substituent, of $A^y$, may include a cyclopropyl group, a cyclobutyl group, a cyclopentyl group, a cyclohexyl group, and a cyclooctyl group.

Examples of the alkynyl group of 2 to 20 carbon atoms in the alkynyl group of 2 to 20 carbon atoms optionally having a substituent, of $A^y$, may include an ethynyl group, a propynyl group, a 2-propynyl group (propargyl group), a butynyl group, a 2-butynyl group, a 3-butynyl group, a pentynyl group, a 2-pentynyl group, a hexynyl group, a 5-hexynyl group, a heptynyl group, an octynyl group, a 2-octynyl group, a nonanyl group, a decanyl group, and a 7-decanyl group.

Examples of the substituents in the alkyl group of 1 to 20 carbon atoms optionally having a substituent and the alkenyl group of 2 to 20 carbon atoms optionally having a substituent, of $A^y$, may include a halogen atom, such as a fluorine atom and a chlorine atom; a cyano group; a substituted amino group, such as a dimethylamino group; an alkoxy group of 1 to 20 carbon atoms, such as a methoxy group, an ethoxy group, an isopropyl group, and a butoxy group; an alkoxy group of 1 to 12 carbon atoms that is substituted by an alkoxy group of 1 to 12 carbon atoms, such as a methoxymethoxy group and a methoxyethoxy group; a nitro group; an aryl group, such as a phenyl group and a naphthyl group; a cycloalkyl group of 3 to 8 carbon atoms, such as a cyclopropyl group, a cyclopentyl group, and a cyclohexyl group; a cycloalkyloxy group of 3 to 8 carbon atoms, such as a cyclopentyloxy group, and a cyclohexyloxy group; a cyclic ether group of 2 to 12 carbon atoms, such as a tetrahydrofuranyl group, a tetrahydropyranyl group, a dioxolanyl group, and a dioxanyl group; an aryloxy group of 6 to 14 carbon atoms, such as a phenoxy group, and a naphthoxy group; a fluoroalkoxy group of 1 to 12 carbon atoms in which at least one is substituted by a fluoro atom, such as a trifluoromethyl group, a pentafluoroethyl group, and $-CH_2CF_3$; a benzofuryl group; a benzopyranyl group; a benzodioxolyl group; a benzodioxanyl group; $-C(=O)-R^{7a}$; $-C(=O)-OR^{7a}$; $-SO_2R^{8a}$; $-SR^{10}$; an alkoxy group of 1 to 12 carbon atoms substituted by $-SR^{10}$; and a hydroxyl group. Herein, $R^{7a}$ and $R^{10}$ are each independently an alkyl group of 1 to 20 carbon atoms, an alkenyl group of 2 to 20 carbon atoms, a cycloalkyl group of 3 to 12 carbon atoms, or an aromatic hydrocarbon group of 6 to 12 carbon atoms. $R^{8a}$ is an alkyl group of 1 to 20 carbon atoms, an alkenyl group of 2 to 20 carbon atoms, a phenyl group, or a 4-methylphenyl group, which are the same as those for $R^4$ described above.

Examples of the substituent in the cycloalkyl group of 3 to 12 carbon atoms optionally having a substituent, of $A^y$, may include a halogen atom, such as a fluorine atom and a chlorine atom; a cyano group; a substituted amino group, such as a dimethylamino group; an alkyl group of 1 to 6 carbon atoms, such as a methyl group, an ethyl group, and a propyl group; an alkoxy group of 1 to 6 carbon atoms, such as a methoxy group, an ethoxy group, and an isopropoxy group; a nitro group; an aryl group, such as a phenyl group and a naphthyl group; a cycloalkyl group of 3 to 8 carbon atoms, such as a cyclopropyl group, a cyclopentyl group, and a cyclohexyl group; $-C(=O)-R^{7a}$; $-C(=O)-OR^{7a}$; $-SO_2R^{8a}$; and a hydroxyl group. Herein, $R^{7a}$ and $R^{8a}$ have the same meanings as described above.

Examples of the substituent in the alkynyl group of 2 to 20 carbon atoms optionally having a substituent, of $A^y$, may include subustituents that are the same as the substituents in the alkyl group of 1 to 20 carbon atoms optionally having a substituent and the alkenyl group of 2 to 20 carbon atoms optionally having a substituent.

In the group represented by $-C(=O)-R^3$ of $A^y$, $R^3$ is an alkyl group of 1 to 20 carbon atoms optionally having a substituent, an alkenyl group of 2 to 20 carbon atoms optionally having a substituent, a cycloalkyl group of 3 to 12 carbon atoms optionally having a substituent, or an aromatic hydrocarbon group of 5 to 12 carbon atoms. Specific examples thereof may include those exemplified as the examples of the alkyl group of 1 to 20 carbon atoms optionally having a substituent, the alkenyl group of 2 to 20 carbon atoms optionally having a substituent, and the cycloalkyl group of 3 to 12 carbon atoms optionally having a substituent, of $A^y$.

In the group represented by $-SO_2-R^4$ of $A^y$, $R^4$ is an alkyl group of 1 to 20 carbon atoms, an alkenyl group of 2 to 20 carbon atoms, a phenyl group, or a 4-methylphenyl group. Specific examples of the alkyl group of 1 to 20 carbon atoms and the alkenyl group of 2 to 20 carbon atoms, of $R^4$, may include those exemplified as the examples of the alkyl group of 1 to 20 carbon atoms, and the alkenyl group of 2 to 20 carbon atoms, of $A^y$ described above.

In the group represented by $-C(=S)NH-R^9$ of $A^y$, $R^9$ is an alkyl group of 1 to 20 carbon atoms optionally having a substituent, an alkenyl group of 2 to 20 carbon atoms optionally having a substituent, a cycloalkyl group of 3 to 12 carbon atoms optionally having a substituent, or an aromatic group of 5 to 20 carbon atoms optionally having a substituent. Specific examples thereof may include those exemplified as the examples of the alkyl group of 1 to 20 carbon atoms optionally having a substituent, the alkenyl group of 2 to 20 carbon atoms optionally having a substituent, and the cycloalkyl group of 3 to 12 carbon atoms optionally having a substituent, of $A^y$ described above.

Examples of the organic group of 2 to 30 carbon atoms having at least one aromatic ring selected from the group consisting of an aromatic hydrocarbon ring and an aromatic heterocyclic ring of $A^y$ may include those exemplified as the examples of $A^x$ exemplified above.

Among these, $A^y$ is preferably a hydrogen atom, an alkyl group of 1 to 20 carbon atoms optionally having a substituent, an alkenyl group of 2 to 20 carbon atoms optionally having a substituent, a cycloalkyl group of 3 to 12 carbon atoms optionally having a substituent, an alkynyl group of 2 to 20 carbon atoms optionally having a substituent, $-C(=O)-R^3$, $SO_2-R^4$, or an organic group of 2 to 30 carbon atoms having at least one aromatic ring selected from the group consisting of an aromatic hydrocarbon ring and an aromatic heterocyclic ring, and further preferably a hydrogen atom, an alkyl group of 1 to 20 carbon atoms optionally having a substituent, an alkenyl group of 2 to 20 carbon atoms optionally having a substituent, a cycloalkyl group of 3 to 12 carbon atoms optionally having a substituent, an alkynyl group of 2 to 20 carbon atoms optionally having a substituent, an aromatic hydrocarbon group of 6 to 12 carbon atoms optionally having a substituent, an aromatic heterocyclic group of 3 to 9 carbon atoms optionally having a substituent, $-C(=O)-R^3$, or a group represented by $-SO_2-R^4$ Herein, $R^3$ and $R^4$ have the same meanings as described above.

It is preferable that substituents in the alkyl group of 1 to 20 carbon atoms optionally having a substituent, the alkenyl group of 2 to 20 carbon atoms optionally having a substituent, and the alkynyl group of 2 to 20 carbon atoms optionally having a substituent, of $A^y$, are a halogen atom, a cyano group, an alkoxy group of 1 to 20 carbon atoms, an alkoxy group of 1 to 12 carbon atoms that is substituted by an alkoxy group of 1 to 12 carbon atoms, a phenyl group, a cyclohexyl group, a cyclic ether group of 2 to 12 carbon atoms, an aryloxy group of 6 to 14 carbon atoms, a hydroxyl group, a benzodioxanyl group, a phenylsulfonyl group, a 4-methylphenylsulfonyl group, a benzoyl group, or $-SR^{10}$. Herein, $R^{10}$ has the same meanings as described above.

It is preferable that substituents in the cycloalkyl group of 3 to 12 carbon atoms optionally having a substituent, the aromatic hydrocarbon group of 6 to 12 carbon atoms optionally having a substituent, and the aromatic heterocyclic group of 3 to 9 carbon atoms optionally having a substituent, of $A^y$, are a fluorine atom, an alkyl group of 1 to 6 carbon atoms, an alkoxy group of 1 to 6 carbon atoms, or a cyano group.

$A^x$ and $A^y$ may form a ring together. Examples of the ring may include an unsaturated heterocyclic ring of 4 to 30 carbon atoms optionally having a substituent and an unsaturated carbon ring of 6 to 30 carbon atoms optionally having a substituent.

The aforementioned unsaturated heterocyclic ring of 4 to 30 carbon atoms and the aforementioned unsaturated carbon ring of 6 to 30 carbon atoms are not particularly restricted, and may or may not have aromaticity.

Examples of the ring formed by $A^x$ and $A^y$ together may include rings shown below. The rings shown below are a moiety of:

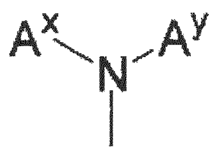

in the formula (I).

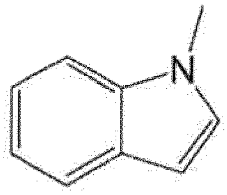
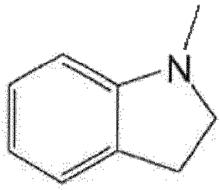
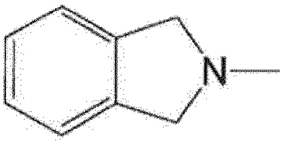
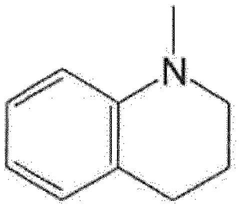

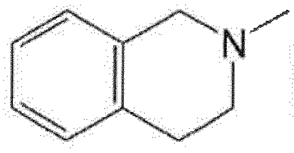
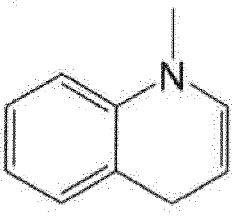
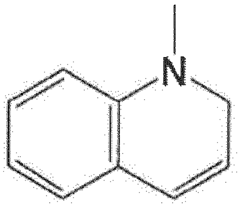
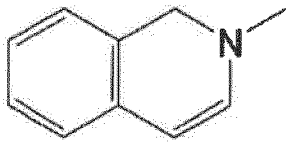

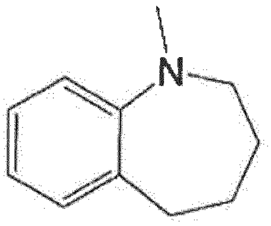
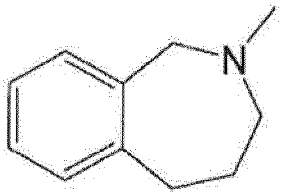
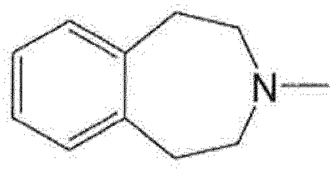

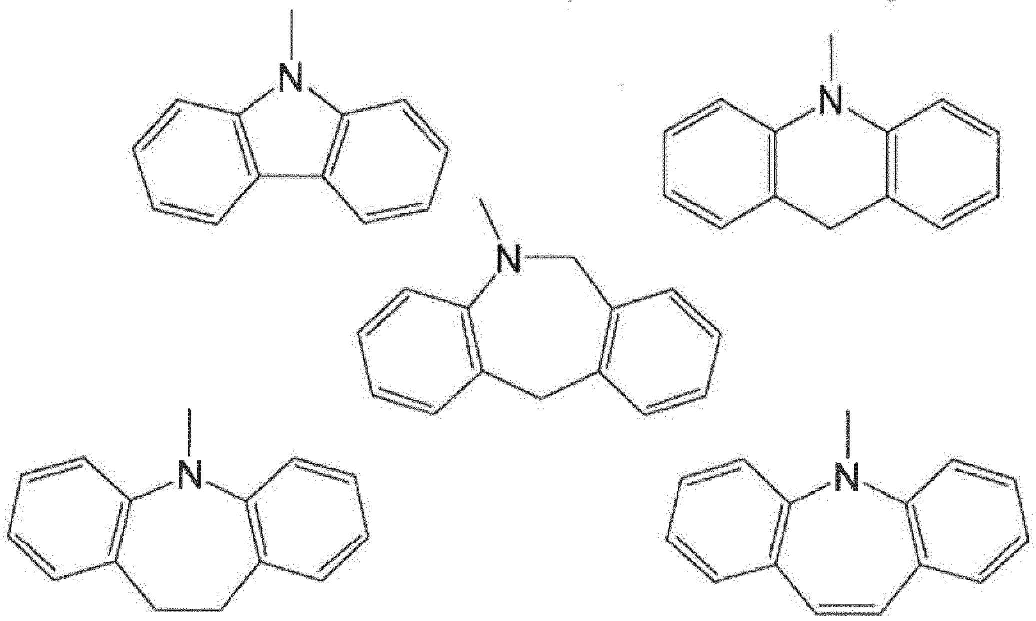

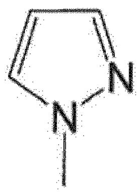
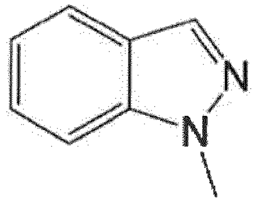
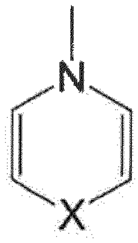
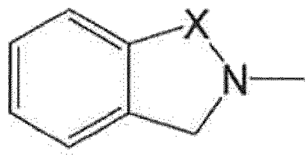
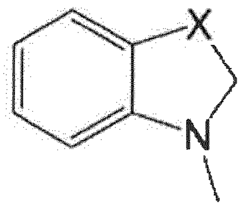
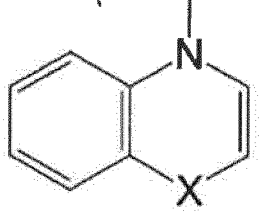
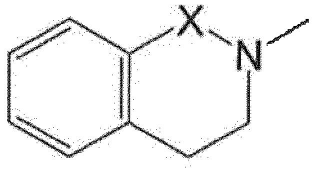
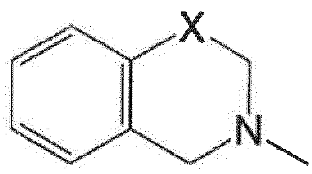
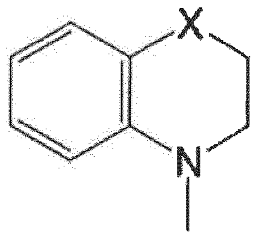
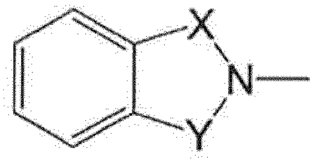
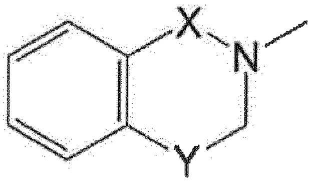
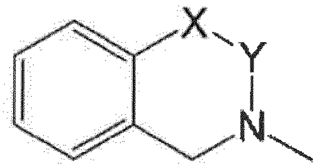
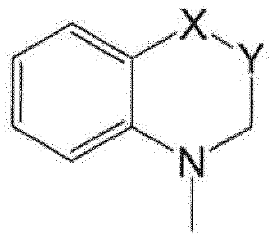
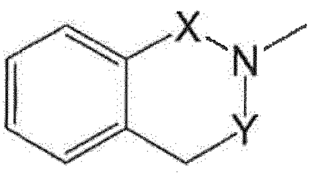
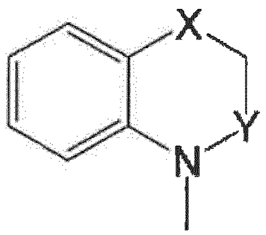
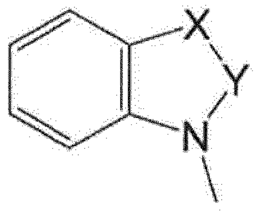
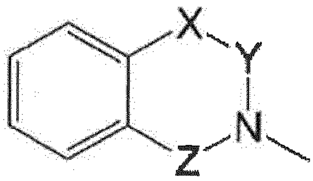
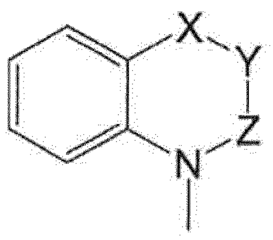

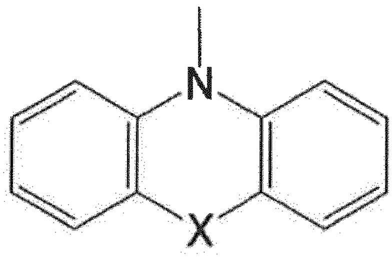
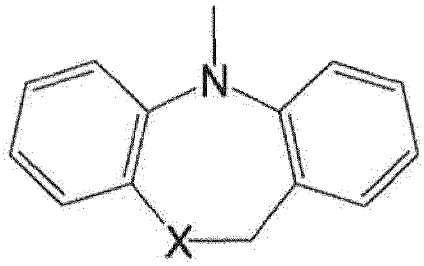
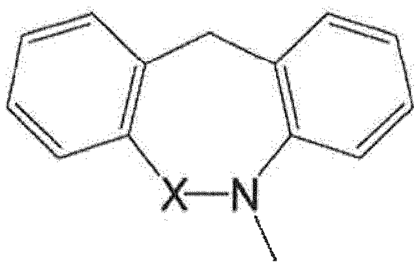
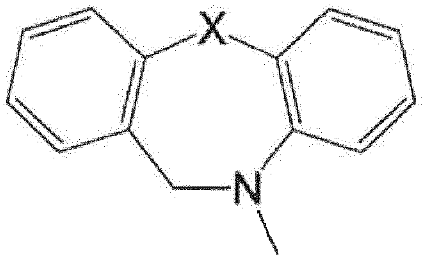
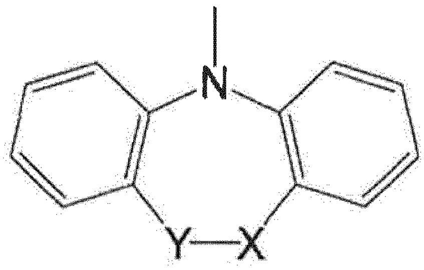
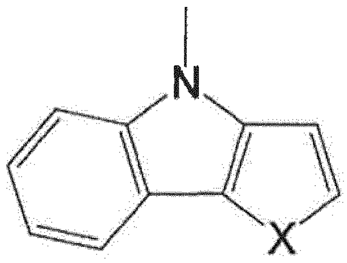
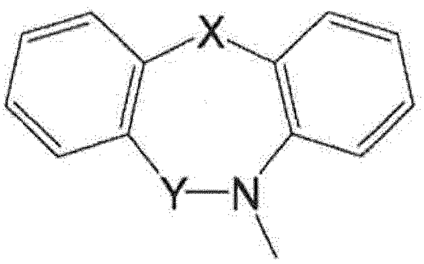
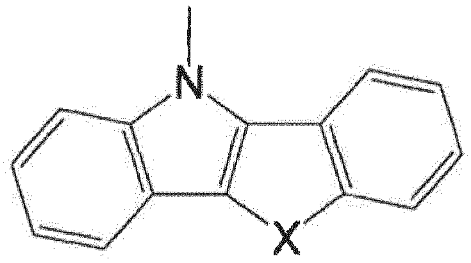

(In the formulae, X, Y, and Z have the same meanings as described above.)

The rings may have a substituent. Examples of the substituent may include those exemplified as the substituent in the aromatic ring of $A^x$.

The total number of $\pi$ electrons contained in $A^x$ and $A^y$ is preferably 4 or more and 24 or less, more preferably 6 or more and 20 or less, and further preferably 6 or more and 18 or less from the viewpoint of favorably expressing the desired effect of the present invention.

Examples of preferred combination of $A^x$ and $A^y$ may include:

($\alpha$) a combination of $A^x$ and $A^y$ in which $A^x$ is an aromatic hydrocarbon group of 4 to 30 carbon atoms or an aromatic heterocyclic group of 4 to 30 carbon atoms, $A^y$ is a hydrogen atom, a cycloalkyl group of 3 to 8 carbon atoms, an aromatic hydrocarbon group of 6 to 12 carbon atoms optionally having a substituent (a halogen atom, a cyano group, an alkyl group of 1 to 6 carbon atoms, an alkoxy group of 1 to 6 carbon atoms, or a cycloalkyl group of 3 to 8 carbon atoms), an aromatic heterocyclic group of 3 to 9 carbon atoms optionally having a substituent (a halogen atom, an alkyl group of 1 to 6 carbon atoms, an alkoxy group of 1 to 6 carbon atoms, or a cyano group), an alkyl group of 1 to 20 carbon atoms optionally having a substituent, an alkenyl group of 1 to 20 carbon atoms optionally having a substituent, or an alkynyl group of 2 to 20 carbon atoms optionally having a substituent, and the substituent is any of a halogen atom, a cyano group, an alkoxy group of 1 to 20 carbon atoms, an alkoxy group of 1 to 12 carbon

atoms that is substituted by an alkoxy group of 1 to 12 carbon atoms, a phenyl group, a cyclohexyl group, a cyclic ether group of 2 to 12 carbon atoms, an aryloxy group of 6 to 14 carbon atoms, a hydroxyl group, a benzodioxanyl group, a benzenesulfonyl group, a benzoyl group, and $-SR^{10}$, and

(β) a combination of $A^x$ and $A^y$ in which $A^x$ and $A^y$ together form an unsaturated heterocyclic ring or an unsaturated carbon ring. Herein, $R^{10}$ has the same meanings as described above.

Examples of more preferred combination of $A^x$ and $A^y$ may include:

(γ) a combination of $A^x$ and $A^y$ in which $A^x$ is any of groups having the following structures, $A^y$ is a hydrogen atom, a cycloalkyl group of 3 to 8 carbon atoms, an aromatic hydrocarbon group of 6 to 12 carbon atoms optionally having a substituent (a halogen atom, a cyano group, an alkyl group of 1 to 6 carbon atoms, an alkoxy group of 1 to 6 carbon atoms, or a cycloalkyl group of 3 to 8 carbon atoms), an aromatic heterocyclic group of 3 to 9 carbon atoms optionally having a substituent (a halogen atom, an alkyl group of 1 to 6 carbon atoms, an alkoxy group of 1 to 6 carbon atoms, or a cyano group), an alkyl group of 1 to 20 carbon atoms optionally having a substituent, an alkenyl group of 1 to 20 carbon atoms optionally having a substituent, or an alkynyl group of 2 to 20 carbon atoms optionally having a substituent, and the substituent is any of a halogen atom, a cyano group, an alkoxy group of 1 to 20 carbon atoms, an alkoxy group of 1 to 12 carbon atoms that is substituted by an alkoxy group of 1 to 12 carbon atoms, a phenyl group, a cyclohexyl group, a cyclic ether group of 2 to 12 carbon atoms, an aryloxy group of 6 to 14 carbon atoms, a hydroxyl group, a benzodioxanyl group, a benzenesulfonyl group, a benzoyl group, and $-SR^{10}$. Herein, $R^{10}$ has the same meanings as described above.

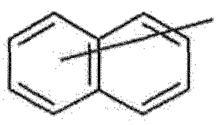
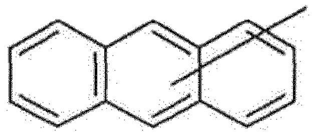

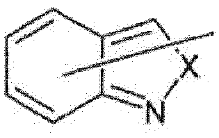
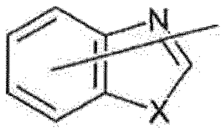
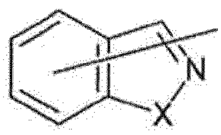
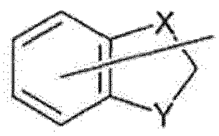

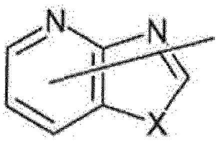
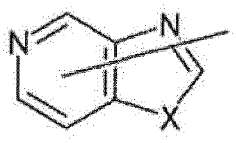
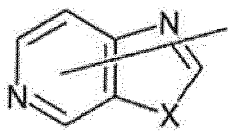

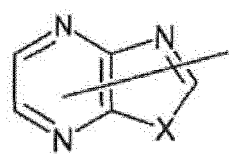
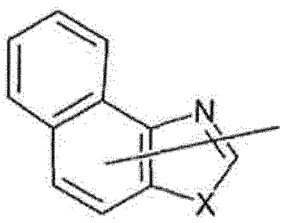
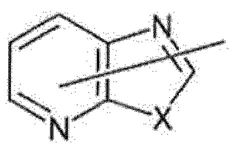

(In the formulae, X and Y have the same meanings as described above.)

Examples of particularly preferred combination of $A^x$ and $A^y$ may include:

(δ) a combination of $A^x$ and $A^y$ in which $A^x$ is any of groups having the following structures, $A^y$ is a hydrogen atom, a cycloalkyl group of 3 to 8 carbon atoms, an aromatic hydrocarbon group of 6 to 12 carbon atoms optionally having a substituent (a halogen atom, a cyano group, an alkyl group of 1 to 6 carbon atoms, an alkoxy group of 1 to 6 carbon atoms, or a cycloalkyl group of 3 to 8 carbon atoms), an aromatic heterocyclic group of 3 to 9 carbon atoms optionally having a substituent (a halogen atom, an alkyl group of 1 to 6 carbon atoms, an alkoxy group of 1 to 6 carbon atoms, or a cyano group), an alkyl group of 1 to 20 carbon atoms optionally having a substituent, an alkenyl group of 1 to 20 carbon atoms optionally having a substituent, or an alkynyl group of 2 to 20 carbon atoms optionally having a substituent, and the substituent is any of a halogen atom, a cyano group, an alkoxy group of 1 to 20 carbon atoms, an alkoxy group of 1 to 12 carbon atoms that is substituted by an alkoxy group of 1 to 12 carbon atoms, a phenyl group, a cyclohexyl

group, a cyclic ether group of 2 to 12 carbon atoms, an aryloxy group of 6 to 14 carbon atoms, a hydroxyl group, a benzodioxanyl group, a benzenesulfonyl group, a benzoyl group, and $-SR^{10}$. In the following formulae, X has the same meanings as described above. Herein, $R^{10}$ has the same meanings as described above.

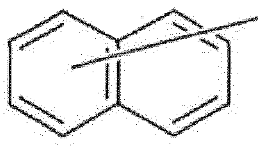

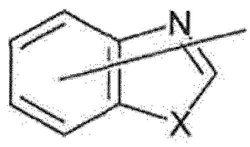

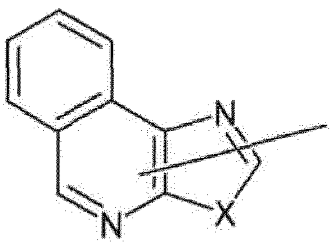

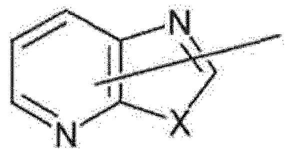

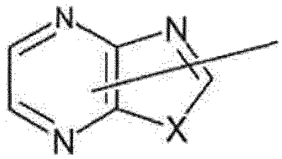

In the formula (I) mentioned above, $A^1$ is a trivalent aromatic group optionally having a substituent. The trivalent aromatic group may be a trivalent carbocyclic aromatic group or a trivalent heterocyclic aromatic group. From the viewpoint of favorably expressing the desired effect of the present invention, the trivalent aromatic group is preferably the trivalent carbocyclic aromatic group, more preferably a trivalent benzene ring group or a trivalent naphthalene ring group, and further preferably a trivalent benzene ring group or a trivalent naphthalene ring group that is represented by the following formula. In the following formulae, substituents $Y^1$ and $Y^2$ are described for the sake of convenience to clearly show a bonding state ($Y^1$ and $Y^2$ have the same meanings as described above, and the same applies to the following).

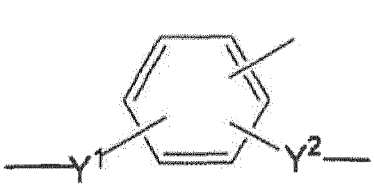

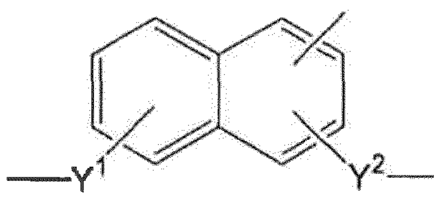

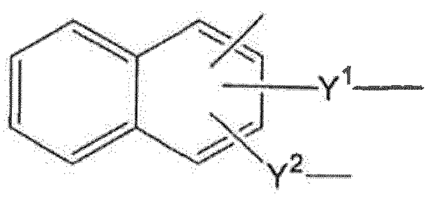

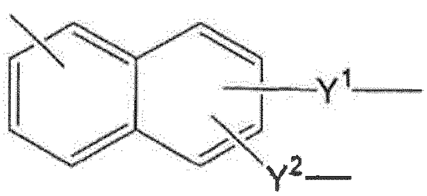

Among these, $A^1$ is more preferably a group represented by each of the following formulae (A11) to (A25), further preferably a group represented by the formula (A11), (A13), (A15), (A19), or (A23), and particularly preferably a group represented by the formula (A11) or (A23).

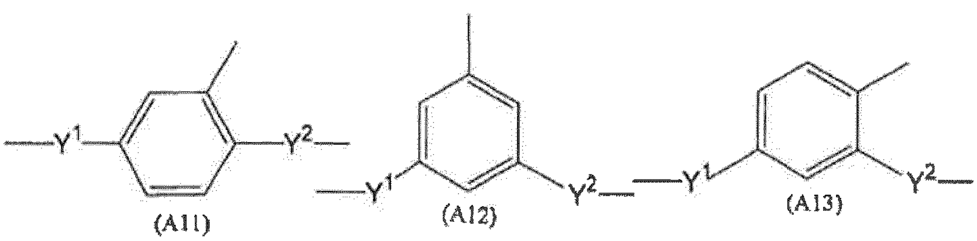

(A11) (A12) (A13)

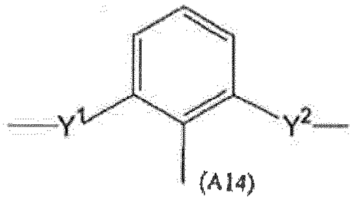
(A14)

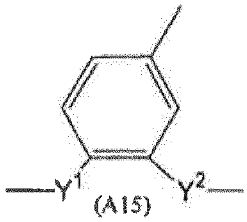
(A15)

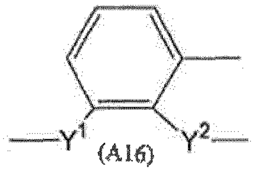
(A16)

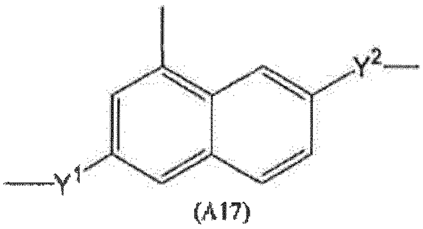
(A17)

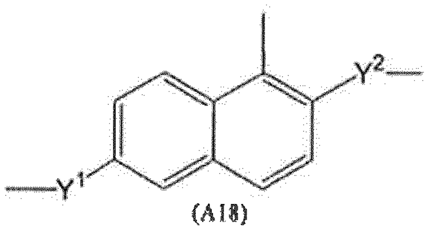
(A18)

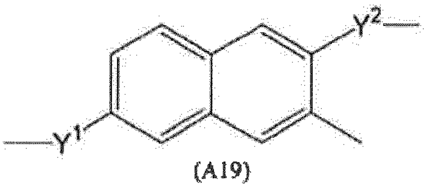
(A19)

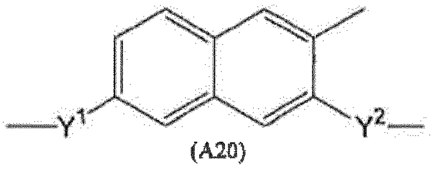
(A20)

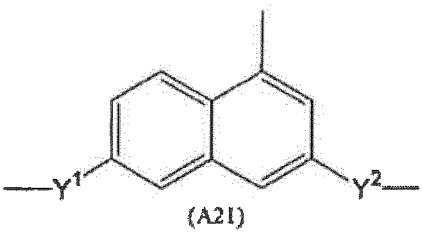
(A21)

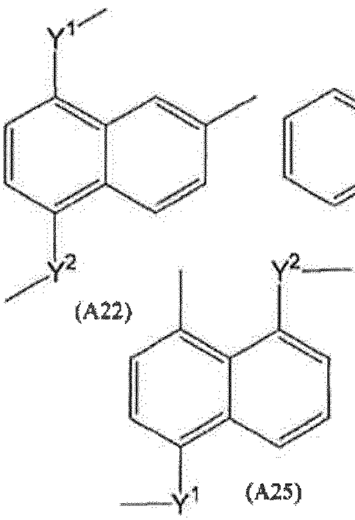
(A22)

(A25)

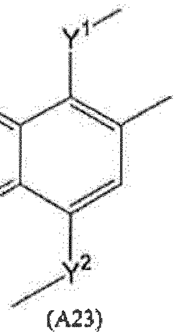
(A23)

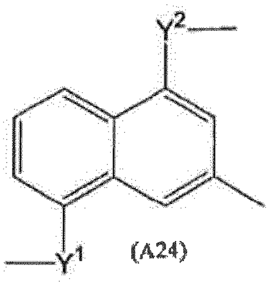
(A24)

Examples of the substituent that may be included in the trivalent aromatic group of $A^1$ may include those exemplified as the substituent in the aromatic group of $A^x$ described above. It is preferable that $A^1$ is a trivalent aromatic group having no substituent.

In the formula (I) mentioned above, $A^2$ and $A^3$ are each independently a divalent alicyclic hydrocarbon group of 3 to 30 carbon atoms optionally having a substituent. Examples of the divalent alicyclic hydrocarbon group of 3 to 30 carbon atoms may include a cycloalkanediyl group of 3 to 30 carbon atoms, and a divalent alicyclic fused ring group of 10 to 30 carbon atoms.

Examples of the cycloalkanediyl group of 3 to 30 carbon atoms may include a cyclopropanediyl group; a cyclobutanediyl group, such as a cyclobutane-1,2-diyl group and a cyclobutane-1,3-diyl group; a cyclopentanediyl group, such as a cyclopentane-1,2-diyl group and a cyclopentane-1,3-diyl group; a cyclohexanediyl group, such as a cyclohexane-1,2-diyl group, a cyclohexane-1,3-diyl group, and a cyclohexane-1,4-diyl group; a cycloheptanediyl group, such as a cycloheptane-1,2-diyl group, a cycloheptane-1,3-diyl group, and a cycloheptane-1,4-diyl group; a cyclooctanediyl group, such as a cyclooctane-1,2-diyl group, a cyclooctane-1,3-diyl group, a cyclooctane-1,4-diyl group, and a cyclooctane-1,5-diyl group; a cyclodecanediyl group, such as a cyclodecane-1,2-diyl group, a cyclodecane-1,3-diyl group, a

cyclodecane-1,4-diyl group, and a cyclodecane-1,5-diyl group; a cyclododecanediyl group, such as a cyclododecane-1,2-diyl group, a cyclododecane-1,3-diyl group, a cyclododecane-1,4-diyl group, and a cyclododecane-1,5-diyl group; a cyclotetradecanediyl group, such as a cyclotetradecane-1,2-diyl group, a cyclotetradecane-1,3-diyl group, a cyclotetradecane-1,4-diyl group, a cyclotetradecane-1,5-diyl group, and a cyclotetradecane-1,7-diyl group; and a cycloeicosanediyl group, such as a cycloeicosane-1,2-diyl group and a cycloeicosane-1,10-diyl group.

Examples of the divalent alicyclic fused ring group of 10 to 30 carbon atoms may include a decalindiyl group, such as a decalin-2,5-diyl group and a decalin-2,7-diyl group; an adamantanediyl group, such as an adamantane-1,2-diyl group and an adamantane-1,3-diyl group; and a bicyclo[2.2.1]heptanediyl group, such as a bicyclo[2.2.1]heptane-2,3-diyl group, a bicyclo[2.2.1]heptane-2,5-diyl group, and a bicyclo[2.2.1]heptane-2,6-diyl group.

The divalent alicyclic hydrocarbon groups may further have a substituent at any position. Examples of the substituent may include those exemplified as the substituent in the aromatic group of $A^x$ described above.

Among these, $A^2$ and $A^3$ are preferably a divalent alicyclic hydrocarbon group of 3 to 12 carbon atoms, more preferably a cycloalkanediyl group of 3 to 12 carbon atoms, further preferably a group represented by each of the following formulae (A31) to (A34), and particularly preferably the group represented by the following formula (A32).

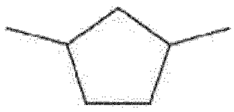
(A31)

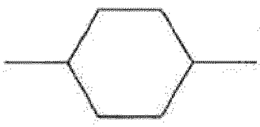
(A32)

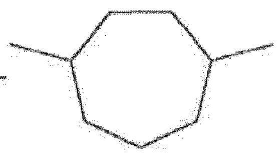
(A33)

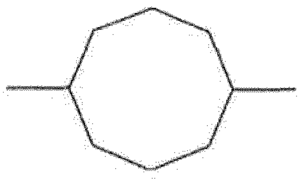
(A34)

The divalent alicyclic hydrocarbon group of 3 to 30 carbon atoms may exist in forms of cis- and transstereoisomers that are on the basis of difference of stereoconfiguration of carbon atoms bonded to $Y^1$ and $Y^3$ (or $Y^2$ and $Y^4$). For example, when the group is a cyclohexane-1,4-diyl group, a cis-isomer (A32a) and a trans-isomer (A32b) may exist, as described below.

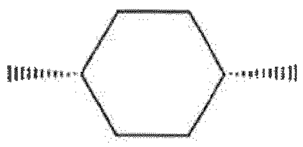
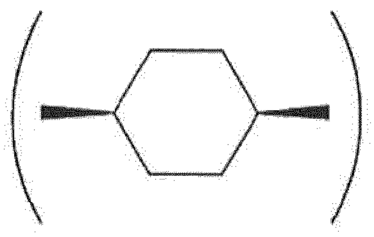
(A32a)

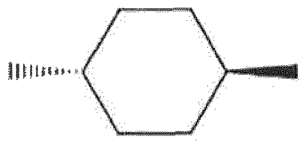
(A32b)

The aforementioned divalent alicyclic hydrocarbon group of 3 to 30 carbon atoms may be a cis-isomer, a trans-isomer, or an isomeric mixture of cis- and trans-isomers. Since the orientation is favorable, the group is preferably the trans-isomer or the cis-isomer, and more preferably the trans-isomer.

In the formula (I) mentioned above, $A^4$ and $A^5$ are each independently a divalent aromatic group of 6 to 30 carbon atoms optionally having a substituent. The aromatic group of $A^4$ and $A^5$ may be monocyclic or polycyclic. Specific preferable examples of $A^4$ and $A^5$ are as follows.

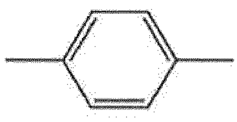
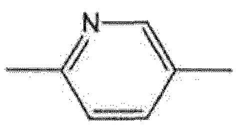
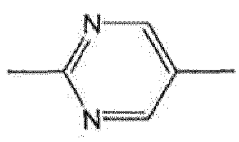
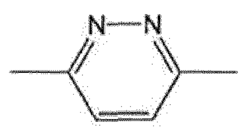

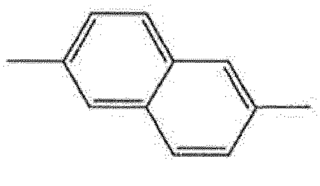
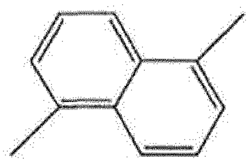
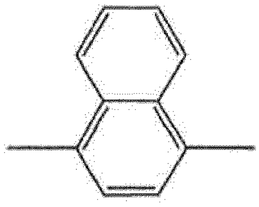

The divalent aromatic groups of $A^4$ and $A^5$ described above may have a substituent at any position. Examples of the substituent may include a halogen atom, a cyano group, a hydroxyl group, an alkyl group of 1 to 6 carbon atoms, an alkoxy group of 1 to 6 carbon atoms, a nitro group, and a -C(=O)-$OR^{8b}$ group. Herein, $R^{8b}$ is an alkyl group of 1 to 6 carbon atoms. In particular, it is preferable that the substituent is a halogen atom, an alkyl group of 1 to 6 carbon atoms, or an alkoxy group. Of the halogen atoms, a fluorine atom is more preferable, of the alkyl groups of 1 to 6 carbon atoms, a methyl group, an ethyl group, and a propyl group are more preferable, and of the alkoxy groups, a methoxy group and an ethoxy group are more preferable.

Among these, from the viewpoint of favorably expressing the desired effect of the present invention, $A^4$ and $A^5$ are each independently preferably a group represented by the following formula (A41), (A42), or (A43) and optionally having a substituent, and particularly preferably the group represented by the formula (A41) and optionally having a substituent.

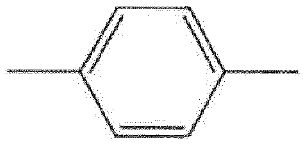
(A41)

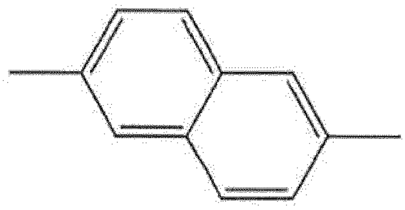
(A42)

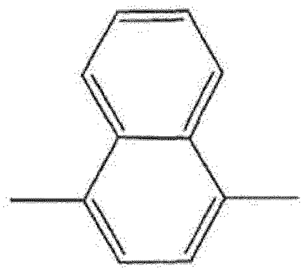
(A43)

In the formula (I) mentioned above, $Q^1$ is a hydrogen atom or an alkyl group of 1 to 6 carbon atoms optionally having a substituent. Examples of the alkyl group of 1 to 6 carbon atoms optionally having a substituent may include those exemplified as to $A^X$ described above. Among these, $Q^1$ is preferably a hydrogen atom or an alkyl group of 1 to 6 carbon atoms, and more preferably a hydrogen atom or a methyl group.

In the formula (I) mentioned above, m and n are each independently 0 or 1. Among these, m is preferably 1, and n is preferably 1.

The refractive index anisotropy $\Delta n$ of the compound (I) is usually small. Specifically, the refractive index anisotropy $\Delta n$ of the compound (I) is preferably 0.1 or less, more preferably 0.08 or less, and particularly preferably 0.07 or less. When the refractive index anisotropy $\Delta n$ of the compound (I) is small as previously described, the selective reflection band of the cholesteric resin layer can be narrowed. The lower limit of the refractive index anisotropy $\Delta n$ of the compound (I) is not limited, but preferably 0.01 or more, more preferably 0.03 or more, and particularly preferably 0.04 or more.

The phase transition temperature from the liquid crystal phase to the isotropic phase of the compound (I) is usually high. Specifically, the phase transition temperature from the liquid crystal phase to the isotropic phase of the

compound (I) is preferably 100°C or higher, more preferably 120°C or higher, and particularly preferably 150°C or higher. When the phase transition temperature from the liquid crystal phase to the isotropic phase of the compound (I) is high in this manner, the compound (I) can maintain the liquid crystal phase (usually the cholesteric phase) when polymerized. Therefore, there can be achieved a cholesteric resin layer having a desired circularly polarized light separation function. The upper limit value of the phase transition temperature from the liquid crystal phase to the isotropic phase of the compound (I) may be any value, but preferably 250°C or lower, more preferably 230°C or lower, and particularly preferably 200°C or lower.

The phase transition temperature from the crystal phase to the liquid crystal phase of the compound (I) is preferably 50°C or higher, more preferably 60°C or higher, and particularly preferably 70°C or higher, and is preferably 140°C or lower, more preferably 130°C or lower, and particularly preferably 120°C or lower.

The molecular weight of the compound (I) is preferably 300 or more, more preferably 700 or more, and particularly preferably 1000 or more, and is preferably 2000 or less, more preferably 1700 or less, and particularly preferably 1500 or less. Such a molecular weight range of the compound (I) indicates that the compound (I) is a monomer. Accordingly, the liquid crystal composition used for forming the cholesteric resin layer can have particularly favorable coating properties.

The compound (I) may be produced by, for example, a reaction between a hydrazine compound and a carbonyl compound described in International Publication No. WO 2014/069515 A1, International Publication No. WO 2015/064581 A1, and International Publication No. WO 2012/147904 A1.

<4.3.2. Polymer Of Polymerizable Liquid Crystal Compound>

The compound (I) may be polymerized to form a layer of the polymer to thereby obtain a cholesteric resin layer. The resulting cholesteric resin layer may be used as the selective reflection layer in the analysis plate according to the present invention. Furthermore, a concavo-convex structure may be formed on the surface of this cholesteric resin layer, to thereby enable formation of the selective reflection layer having a concavo-convex structure.

The polymer contained in the cholesteric resin layer may be a copolymer of the compound (I) and an optional monomer that is polymerizable with the compound (I), but preferably a polymer of only the compound (I). When the polymer is a polymer of only the compound (I), the polymer may be a homopolymer of one type of the compound (I), or may be a copolymer of two or more types of the compounds (I). This polymer may be crosslinked.

The polymer contained in the cholesteric resin layer has cholesteric regularity. As described herein, the "cholesteric regularity" is a structure in which the angles of molecular axes in stacking planes are shifted (twisted) as the planes are observed sequentially passing through the stacked planes, such that molecular axes in a first plane are oriented in a certain direction, molecular axes in a subsequent plain stacking on the first plane are oriented in a direction shifted by a small angle with respect to that of the first plane, and molecular axes in still another plane are oriented in a direction of a further shifted angle. The structure in which the direction of molecular axes is continuously twisted in this manner is usually a helical structure, and is thus an optically chiral structure. It is preferable that the normal line (helical axis) of the plane is approximately parallel to the thickness direction of the cholesteric resin layer.

The cholesteric resin layer containing a polymer having cholesteric regularity as previously described usually has a circularly polarized light separation function. Therefore, when light enters the cholesteric resin layer, only one of counter-clockwise circularly polarized light and clockwise circularly polarized light in a specific wavelength region is reflected, and light other than the reflected circularly polarized light passes through the cholesteric resin layer. This wavelength region in which circularly polarized light is reflected is the selective reflection band.

The specific wavelength in which the cholesteric resin layer containing a polymer of a polymerizable liquid crystal compound exerts a circularly polarized light separation function usually depends on the pitch of the helical structure of the polymer in the cholesteric resin layer. Therefore, the wavelength in which the circularly polarized light separation function is exerted may be controlled by adjusting the size of the pitch of this helical structure.

Further specifically, if the helical axis representing the rotation axis when the molecular axis is twisted in the helical structure is parallel to the normal line of the cholesteric resin layer, a pitch length p of the helical structure and a wavelength $\lambda$ of the circularly polarized light to be reflected have a relationship of formula (X) and formula (Y). As described herein, the pitch length p of the helical structure is a distance in the plane normal line direction, from when the angle of the molecular axis direction starts gradually twisted in a continuous manner as proceeding on planes in the helical structure, to when the angle returns to the original molecular axis direction.

$$\text{Formula (X): } \lambda_c = n \times p \times \cos\theta$$

$$\text{Formula (Y): } n_o \times p \times \cos\theta \leq \lambda \leq n_e \times p \times \cos\theta$$

In the formula (X) and the formula (Y), $\lambda_c$ represents the center wavelength of the selective reflection band, $n_o$ represents the refractive index in the minor axial direction of the polymerizable liquid crystal compound, $n_e$ represents the refractive index in the major axis direction of the polymerizable liquid crystal compound, n represents $(n_e + n_o)/2$, p represents the pitch length of the helical structure, and $\theta$ represents an incident angle (an angle formed with the normal line of the plane) of light.

Therefore, the center wavelength $\lambda_c$ of the selective reflection band depends on the pitch length p of the helical structure of the polymer in the cholesteric resin layer. The selective reflection band may be changed by changing this pitch length p of the helical structure. Therefore, it is preferable that the pitch length p of the helical structure of the polymer is set depending on the wavelength of circularly polarized light desired to be reflected on the selective reflection layer. An example of the method for adjusting the pitch length p may be a publicly known method disclosed in Japanese Patent Application Laid-Open No. 2009-300662 A. Specific examples thereof may include a method of selecting the type of a chiral agent and a method of adjusting the amount of the chiral agent.

Furthermore, the bandwidth of the selective reflection band depends on a difference between the refractive index $n_e$ in the major axial direction and the refractive index $n_o$ in the minor axis direction of the polymerizable liquid crystal compound, and therefore depends on the refractive index anisotropy $\Delta n$ of the polymerizable liquid crystal compound. Thus, the bandwidth of the selective reflection band of the cholesteric resin layer may be narrowed by using the compound (I) having a small refractive index anisotropy $\Delta n$ as the polymerizable liquid crystal compound.

<4.3.3. Optional Component Of Cholesteric Resin Layer>

The cholesteric resin layer may include an optional component in addition to the aforementioned polymer of the compound (I). As such an optional component, a component which does not significantly impair the circularly polarized light separation function of the cholesteric resin layer is preferable. For example, the cholesteric resin layer may contain a surfactant which may be contained in the liquid crystal composition used for forming the cholesteric resin layer. As the optional component, one type thereof may be solely used, and two or more types thereof may also be used in combination at any ratio.

<4.3.4. Optical Properties Of Cholesteric Resin Layer>

As previously described, since the cholesteric resin layer contains a polymer having cholesteric regularity, it has a selective reflection band in which circularly polarized light can be reflected. Since the compound (I), which is a monomer of a polymer contained in a cholesteric resin layer, has a small refractive index anisotropy $\Delta n$, the selective reflection band of the cholesteric resin layer is usually narrow. The specific bandwidth of the selective reflection band of the cholesteric resin layer may be set depending on the optical properties required of the selective reflection layer. Specifically, the bandwidth may be appropriately set such that the half width $W_2$ of the band of the light reflection caused by selective reflectivity falls within a preferable range in terms of the aforementioned relationship with the half width $W_1$ of the band of the light reflection caused by the concavo-convex structure. More specifically, the half width of the selective reflection band of the cholesteric resin layer is preferably 80 nm or less, more preferably 50 nm or less, and particularly preferably 30 nm or less. The lower limit value of the half width of the selective reflection band is not particularly limited, but may be preferably 15 nm or more, more preferably 20 nm or more, and particularly preferably 25 nm or more.

The half width of the selective reflection band of the cholesteric resin layer may be measured using a spectral transmission meter.

The specific wavelength of the selective reflection band of the cholesteric resin layer may be set depending on the optical properties required of the selective reflection layer. For example, the selective reflection band of the cholesteric resin layer may be within the visible range (wavelength: 400 nm or more and 800 nm or less), within the infrared range (wavelength: more than 800 nm), or within the ultraviolet range (wavelength: not less than 1 nm and less than 400 nm). From the viewpoint of performing favorable measurement of a measurement target substance, the selective reflection band is usually set in the visible range, more preferably in the green region (wavelength: 495 nm or more and 570 nm or less).

<4.3.5. Thickness Of Cholesteric Resin Layer>

The thickness per layer of the cholesteric resin layer in the substrate is preferably 0.5 $\mu$m or more, more preferably 1.5 $\mu$m or more, and particularly preferably 3.0 $\mu$m or more, and is preferably 12 $\mu$m or less, more preferably 10 $\mu$m or less, and particularly preferably 8 $\mu$m or less. When the thickness of the cholesteric resin layer is equal to or more than the lower limit value of the aforementioned range, circularly polarized light in the selective reflection band can be effectively reflected. When the thickness thereof is equal to or less than the upper limit value of the aforementioned range, the transmittance of light in the wavelength band other than the selective reflection band can be enhanced.

<4.3.6. Formation Of Selective Reflection Layer With Polymerizable Liquid Crystal Compound>

The selective reflection layer may be produced by a production method which includes: a step of forming a layer of a liquid crystal composition containing a polymerizable liquid crystal compound such as the compound (I) on an appropriate support; and a step of polymerizing the polymerizable liquid crystal compound contained in the layer of the liquid crystal composition.

Hereinafter, an example of a general method for forming the selective reflection layer with the compound (I) as the polymerizable liquid crystal compound will be firstly specifically described. Thereafter, a specific method for forming the selective reflection layer including semi-curing and full-curing steps will be described.

<4.3.6.1. Preparation Of Support>

As the support, a film-shaped member is usually used. As the material of the support, a resin may be used. This resin contains a polymer and, as necessary, an optional component. Examples of the polymer contained in the resin may include those that are the same as the examples of the material of a layer other than the layer having a concavo-convex structure (for example, the support layer 412 in the example illustrated in FIG. 4). The support having been used for forming the selective reflection layer may be used as it is as a support constituting the analysis plate according to the present invention without being peeled from the selective reflection layer.

The support may be a single-layer film including only one layer, or may be a multi-layer film including two or more layers. From the viewpoint of productivity and costs, a single-layer structure film is preferably used as the support. From the viewpoint of favorably orienting the compound (I) during the formation of the cholesteric resin layer, the support may be a multi-layer film having an orientation film.

For example, the orientation film may be formed with a resin which contains a polymer such as polyimide, polyvinyl alcohol, polyester, polyarylate, polyamide imide, polyether imide, and polyamide. As these polymers, one type thereof may be solely used, and two or more types thereof may also be used in combination at any ratio. The orientation film may be produced by applying a solution containing the aforementioned polymer, drying the solution, and performing a rubbing treatment. The thickness of the orientation film is preferably 0.01 μm or more, and more preferably 0.05 μm or more, and is preferably 5 μm or less, and more preferably 1 μm or less.

The support may be the one which has been subjected to a surface treatment on one surface or both surfaces thereof. By performing a surface treatment, adhesion with another layer directly formed on the surface of the support can be improved. Usually, a surface of the support on which a layer of the liquid crystal composition is to be formed is subjected to the surface treatment. Examples of the surface treatment may include an energy ray irradiation treatment and a chemical treatment.

Furthermore, the surface of the support on which a layer of the liquid crystal composition is to be formed may be subjected to a rubbing treatment, in order to promote the orientation of the compound (I) during the formation of the cholesteric resin layer.

The thickness of the support is preferably 30 μm or more, and more preferably 60 μm or more, and is preferably 300 μm or less, and more preferably 200 μm or less, from the viewpoint of handling properties during production, costs of the material, and reduction in thickness and weight.

<4.3.6.2. Step Of Forming Layer Of Liquid Crystal Composition>

After the support has been prepared, a step of forming a layer of a liquid crystal composition containing the compound (I) on the support is performed. The liquid crystal composition is a composition which contains at least the compound (I) and is usually a composition in a fluid state in the step of forming a layer of the liquid crystal composition on the support.

The liquid crystal composition may contain a chiral agent in combination with the compound (I). The twisting direction of the polymer contained in the cholesteric resin layer may be selected by selecting the type and structure of the chiral agent. Examples of the chiral agent may include those disclosed in Japanese Patent Application Laid-Open No. 2003-66214 A, Japanese Patent Application Laid-Open No. 2003-313187 A, U.S. Patent No. 6,468,444 B1, International Publication No. WO 98/00428 A1, and the like. Among these, the chiral agent having a large HTP is preferable from the viewpoint of economy. The HTP is an index indicating efficiency with which the polymerizable liquid crystal compound is twisted. The chiral agent may or may not exhibit liquid crystal properties. Furthermore, from the viewpoint of increasing the crosslinking degree with the polymerizable liquid crystal compound to stabilize the polymer, the chiral agent having a polymerizable group is preferable. As the chiral agent, one type thereof may be solely used, and two or more types thereof may also be used in combination at any ratio.

The amount of the chiral agent is preferably 0.01 parts by weight or more, more preferably 0.1 parts by weight or more, and particularly preferably 0.5 parts by weight or more, and is preferably 35 parts by weight or less, more

preferably 25 parts by weight or less, and particularly preferably 15 parts by weight or less, relative to 100 parts by weight of the compound (I). When the amount of the chiral agent falls within the aforementioned range, cholesteric regularity may be expressed in the compound (I) without lowering crystal liquid properties in a layer of the liquid crystal composition formed on the support.

The liquid crystal composition may contain a polymerization initiator in combination with the compound (I). As the polymerization initiator, there may be used either a thermal polymerization initiator or a photopolymerization initiator. Of these, the photopolymerization initiator is preferable, because therewith polymerization can be promoted more easily and efficiently. Examples of the photopolymerization initiator may include a polynuclear quinone compound (U.S. Patent No. 3,046,127 A and U.S. Patent No. 2,951,758 A), an oxadiazole compound (U.S. Patent No. 4,212,970 A), an $\alpha$-carbonyl compound (U.S. Patent No. 2,367,661 A, U.S. Patent No. 2,367,670 A), acyloin ether (U.S. Patent No. 2,448,828 A), an $\alpha$-hydrocarbon substituted aromatic acyloin compound (U.S. Patent No. 2,722,512 A), a combination of a triaryl imidazole dimer and p-aminophenyl ketone (U.S. Patent No. 3,549,367 A), and acridine and a phenazine compound (Japanese Patent Application Laid-Open No. Sho. 60-105667 A, U.S. Patent No. 4,239,850A). As the polymerization initiator, one type thereof may be solely used, and two or more types thereof may also be used in combination at any ratio.

The amount of the polymerization initiator is preferably 1 part by weight or more, and is preferably 10 parts by weight or less, and more preferably 5 parts by weight or less, relative to 100 parts by weight of the compound (I).

The liquid crystal composition may contain a surfactant in combination with the compound (I). When the surfactant is used, the surface tension of the layer of the liquid crystal composition may be adjusted. The surfactant is preferably a nonionic surfactant, and is preferably an oligomer having a molecular weight of roughly several thousands. As the surfactant, one type thereof may be solely used, and two or more types thereof may also be used in combination at any ratio.

The amount of the surfactant is preferably 0.01 parts by weight or more, more preferably 0.03 parts by weight or more, and particularly preferably 0.05 parts by weight or more, and is preferably 10 parts by weight or less, more preferably 5 parts by weight or less, and particularly preferably 1 part by weight or less, relative to 100 parts by weight of the compound (I). When the amount of the surfactant falls within the aforementioned range, there may be formed a cholesteric resin layer which does not have orientation defects.

The liquid crystal composition may contain a solvent in combination with the compound (I). Examples of the solvent may include an organic solvent such as a ketone solvent, an alkyl halide solvent, an amide solvent, a sulfoxide solvent, a heterocyclic compound, a hydrocarbon solvent, an ester solvent, and an ether solvent. Among these, a ketone solvent is preferable, in consideration of a load on the environment. As the solvent, one type thereof may be solely used, and two or more types thereof may also be used in combination at any ratio.

The amount of the solvent is preferably 40 parts by weight or more, more preferably 60 parts by weight or more, and particularly preferably 80 parts by weight or more, and is preferably 1000 parts by weight or less, more preferably 800 parts by weight or less, and particularly preferably 600 parts by weight or less, relative to 100 parts by weight of the compound (I). When the amount of the solvent falls within the aforementioned range, occurrence of coating unevenness during the coating with the liquid crystal composition can be suppressed, so that a liquid crystal composition layer having a uniform thickness can be formed.

The liquid crystal composition may further contain an optional component as necessary. Examples of the optional component may include: a polymerization inhibiting agent for improving pot life; a crosslinking agent for enhancing the mechanical strength of the cholesteric resin layer; and an antioxidant, a UV absorber, and a photo-stabilizer for improving durability. As the optional component, one type thereof may be solely used, and two or more types thereof may also be used in combination at any ratio. The amount of the optional component may be optionally set within the range that does not reduce desired optical performance.

Usually, a layer of the liquid crystal composition is formed by applying the liquid crystal composition onto the support. Examples of the coating method may include a spin coating method, a roll coating method, a flow coating method, a printing method, a dip coating method, a flow casting method, a bar coating method, a die coating method, and a gravure printing method.

<4.3.6.3. Drying Step>

After the layer of the liquid crystal composition has been formed on the support, a step of drying the layer of the liquid crystal composition may be performed as necessary. The drying method may be any method. The temperature condition during drying may be set to fall within a range of 40°C to 150°C, for example.

<4.3.6.4. Orientation Step>

After the layer of the liquid crystal composition has been formed on the support, a step of subjecting it to an orientation treatment may be performed as necessary. The orientation treatment may be performed by, for example,

heating at 50°C to 150°C for 0.5 to 10 minutes. When the layer of the liquid crystal composition is subjected to the orientation treatment, orientation of the compound (I) contained in the layer can be promoted. Accordingly, the compound (I) can become in the state of a liquid crystal phase having cholesteric regularity.

<4.3.6.5. Polymerization Step>

After the layer of the liquid crystal composition has been formed on the support, a step of polymerizing the polymerizable liquid crystal compound contained in the layer of the liquid crystal composition is performed. Consequently, the polymerization is performed while the cholesteric regularity of the compound (I) is maintained. Therefore, a cholesteric resin layer as the selective reflection layer can be obtained.

The method for polymerizing the compound (I) may be any method. For example, when a photopolymerization initiator is used as the polymerization initiator, polymerization of the compound (I) may be achieved by irradiation of the layer of the liquid crystal composition with light. The light for the irradiation may include not only visible light but also UV light and other electromagnetic waves. Usually, the irradiation is performed with UV light. The irradiation energy of light is preferably 300 $mJ/cm^2$ or more, more preferably 1000 $mJ/cm^2$ or more, and particularly preferably 2000 $mJ/cm^2$ or more, and is preferably 7000 $J/cm^2$ or less, more preferably 6000 $J/cm^2$ or less, and particularly preferably 5000 $J/cm^2$ or less. The light irradiation may be performed to the side onto which the polymerizable liquid crystal composition has been applied (the side of the layer of the liquid crystal composition), or may be performed to the support.

<4.3.6.6. Forming Method Including Semi-Curing And Full-Curing Steps>

When the substrate to be produced is a substrate in which the layer positioned on the surface thereof having a concavo-convex structure is the selective reflection layer, and the selective reflection layer is a cholesteric resin layer, as in the examples of FIG. 1 and FIG. 4, the selective reflection layer is preferably formed by the following forming method including semi-curing and full-curing steps. Specifically, the selective reflection layer is preferably formed by a method including the following steps of (a) to (d).

Step (a): forming a layer of a liquid crystal composition containing a polymerizable liquid crystal compound capable of exhibiting a cholesteric liquid crystal phase.
Step (b): semi-curing the layer of the liquid crystal composition to obtain a semi-cured layer.
Step (c): forming a concavo-convex structure capable of exhibiting a structural color on the surface of the semi-cured layer.
Step (d): fully-curing the semi-cured layer to obtain a substrate.

The step (a) may be performed in the same manner as that in the forming method of a layer of the liquid crystal composition in the aforementioned general forming method of the selective reflection layer. After the completion of the step (a), one or both of the drying step and the orientation step for the liquid crystal composition may be performed as necessary at any stage before the completion of the step (d). The specific operation of these steps may be performed in the same manner as that in the aforementioned general forming method of the selective reflection layer.

The semi-curing in the step (b) refers to an action of performing polymerization to a stage at which the polymerization conversion ratio of the polymerizable liquid crystal compound in a final product is partly achieved. By performing semi-curing, there can be formed a layer in a state in which, for example, even when the layer is pressed at a light pressure with a fingertip, fingerprints do not remain and stickiness is not shown.

The polymerization conversion ratio achieved by the polymerization through semi-curing may be preferably 50% to 80% of the polymerization conversion ratio achieved by full-curing. For example, when the polymerization conversion ratio achieved by full-curing is 90%, the polymerization conversion ratio achieved by the polymerization through semi-curing may be 45% to 72%.

The specific method for semi-curing may be any method. Specifically, semi-curing may be achieved by performing the light irradiation step in the aforementioned general forming method of the selective reflection layer with the irradiation energy quantity changed.

The irradiation energy of light (integrated light quantity) is preferably 1 to 1000 $mJ/cm^2$, and more preferably 10 to 200 $mJ/cm^2$. The illuminance of light is preferably 1 $mW/cm^2$ or more and 500 $mW/cm^2$ or less, and more preferably 1 $mW/cm^2$ or more and 200 $mW/cm^2$ or less.

The step (c) may be performed by bringing a mold such as a metal mold having a reversed shape of a desired concavo-convex structure into pressure contact with the semi-cured layer to transfer the concavo-convex structure thereonto. Since the semi-cured layer is softer than the fully-cured layer, the concavo-convex structure can be easily transferred onto a semi-cured layer.

The mold may be produced by any known method. The material properties and the like of the mold are not

particularly limited, and any known material may be used. A specific example of the mold may be an emboss roll.

The pressure by which the mold is in pressure contact with the semi-cured layer is preferably 0.5 to 50 MPa, and more preferably 1 to 30 MPa.

For more efficiently transferring the concavo-convex structure, it is preferable that the transfer is performed with the mold while the semi-cured layer is heated. The transfer temperature is preferably 50 to 200°C, and more preferably 70 to 110°C. When the transfer temperature falls within the aforementioned range, the phase transition from the liquid crystal phase to the isotropic phase of the polymerizable liquid crystal compound can be suppressed.

When the formation of a concavo-convex structure on the surface of the semi-cured layer is performed using an emboss roll, the layer of the liquid crystal composition is moved while the concavo-convex structure is transferred onto the layer of the liquid crystal composition by the emboss roll.

The moving rate of the layer of the liquid crystal composition is preferably 1 to 50 m/min, and more preferably 3 to 20 m/min.

By performing the step (d) after the step (c), the polymerizable liquid crystal compound is polymerized while maintaining cholesteric regularity. Accordingly, there can be obtained a cholesteric resin layer having a concavo-convex structure on the surface thereof.

The full-curing in the step (d) refers to action of performing polymerization until the polymerization conversion ratio of the polymerizable liquid crystal compound in a final product is achieved. The polymerization conversion ratio in the step (d) may be 85% to 100%.

The specific method for full-curing may be any method. Specifically, full-curing may be achieved by the same operation as that in the light irradiation step in the aforementioned general forming method of the selective reflection layer. The irradiation energy of light (integrated light quantity) and other conditions in the step (d) may be the same as those in the light irradiation step in the aforementioned general forming method of the selective reflection layer, or may be appropriately changed as necessary. Specifically, the irradiation energy (integrated light quantity) is preferably 1 to 5000 mJ/cm$^2$, and more preferably 100 to 3000 mJ/cm$^2$. The light illuminance is preferably 1 mW/cm$^2$ or more and 5000 mW/cm$^2$ or less, and more preferably 100 mW/cm$^2$ or more and 3000 mW/cm$^2$ or less.

The step (c) and the step (d) may be performed in this order, but may be simultaneously performed. When they are simultaneously performed, the concavo-convex structure is transferred with a mold while UV irradiation is performed for full-curing.

By the method including the steps (a) to (d), there can be obtained, on the support, the selective reflection layer having the concavo-convex structure. Thereafter, an optional operation such as peeling the support and bonding to an optional layer may be performed as necessary to obtain the substrate. Further, by performing the aforementioned step of immobilizing a specific recognition molecule on the surface of the resulting substrate, the analysis plate according to the present invention may be obtained.

## Examples

Hereinafter, the present invention will be specifically described with reference to Examples. However, the present invention is not limited to the following Examples. The present invention may be freely modified for implementation without departing from the scope of claims. Unless otherwise specified, "%" and "part(s)" that represent an amount in the following description are on the basis of weight. Unless otherwise specified, operations described below were performed under conditions of normal temperature and normal pressure in atmospheric air.

<Example 1>

A liquid crystal composition was obtained by mixing: 1 part of a polymerizable liquid crystal compound represented by the following formula (A) (refractive index anisotropy Δn = 0.07, phase transition temperature from liquid crystal phase to isotropic phase: 200°C or higher, phase transition temperature from crystal phase to liquid crystal phase: 102°C); 0.13 part of a chiral agent ("LC756" manufactured by BASF Co., Ltd.); 0.035 parts of a photopolymerization initiator ("IRGACURE® 379" manufactured by BASF Co., Ltd.); 0.0013 parts of a surfactant ("s242" manufactured by AGC Seimi Chemical Co., Ltd.); and 1.5 parts of cyclopentanone as a solvent.

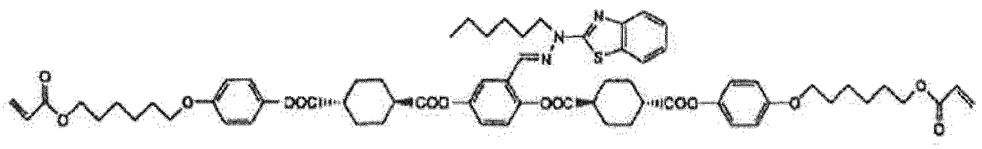

(A)

As a support film, a polyethylene terephthalate film (thickness: 100 μm) of which one surface was subjected to an adhesion facilitating treatment was prepared. A surface of this support film which was not subjected to an adhesion facilitating treatment was subjected to a rubbing treatment. Subsequently, onto the surface having been subjected to a rubbing treatment, the aforementioned liquid crystal composition was applied. Accordingly, a layer of the liquid crystal composition in an uncured state was formed on one surface of the support film.

Thereafter, drying was performed under the conditions of 80°C and 1 minute to remove the solvent from the layer of the liquid crystal composition. Furthermore, an orientation treatment was performed under the conditions of 130°C and 2 minutes to orient the polymerizable liquid crystal compound.

Subsequently, the layer of the liquid crystal composition was irradiated with UV light at an illuminance of 100 $mW/cm^2$ and an integrated light quantity of 100 $mJ/cm^2$ in the air atmosphere, so that the layer of the liquid crystal composition was semi-cured. Thus, a semi-cured layer was formed.

There was prepared an emboss roll on which a concavo-convex structure (diffraction grating pattern) having an emboss shape with a depth of 0.3 μm and a pitch of approximately 8 μm was formed. The temperature of the emboss roll was set at 100°C. The semi-cured layer was pressed with a pressure of 5 MPa using the emboss roll while the semi-cured layer was moved at a rate of 10 m/min. Thus, the concavo-convex structure was formed on the semi-cured layer.

The semi-cured layer on which the concavo-convex structure was formed was irradiated with UV light at an illuminance of 1000 $mW/cm^2$ and an integrated light quantity of 2000 $mJ/cm^2$ under nitrogen atmosphere for full-curing. By the UV irradiation, polymerization of the polymerizable liquid crystal compound proceeded for full-curing. Thus, the cholesteric resin layer (thickness: 5 μm) having a concavo-convex structure schematically illustrated in FIG. 2 was obtained. The concavo-convex structure on the surface of the resulting cholesteric resin layer had recesses periodically laid out in two directions that are a direction along the line L1 and a direction along the line L2 orthogonal to the line L1, as illustrated in FIG. 2. The repeating period P1 in the direction along the line L1 was 8 μm, and the repeating period P2 in the direction along the line L2 was 8 μm.

A specific recognition molecule was immobilized on the surface having this concavo-convex structure of the resulting cholesteric resin layer that served as the substrate. As the specific recognition molecule, an antibody (anti-human fibrinogen antibody) solution (1 μg/mL) was prepared. This solution was dropped on the surface having the concavo-convex structure, and left to stand at room temperature for 1 hour. By this operation, physical adsorption of the antibody on the surface by a hydrophobic interaction was achieved, to thereby immobilize the antibody on the surface. Thus, an analysis plate was obtained.

The color tone of the surface having the concavo-convex structure of the resulting analysis plate was measured using a spectrometer (USB4000 manufactured by Ocean Optics, Inc. or "TECAN" manufactured by Wako Pure Chemical Industries, Ltd.). As a result, reflection having the peak at a wavelength of 550 nm was observed. Subsequently, an analyte (containing human fibrinogen) solution (1 μg/mL) was dropped on the surface, and left to stand at room temperature for 1 hour. The resultant product was washed with ultrapure water, and dried. Then, the color tone on the surface was measured again. As a result, the reflection intensity of the peak at the wavelength of 550 nm decreased by approximately 20% compared to that before dropping the analyte. Thus, a decrease in reflection due to binding to the antigen was clearly observed.

<Example 2>

A liquid crystal composition was obtained by mixing: 1 part of a polymerizable liquid crystal compound represented by the following formula (A) (refractive index anisotropy Δn = 0.07, phase transition temperature from liquid crystal phase to isotropic phase: 200°C or higher, phase transition temperature from crystal phase to liquid crystal phase: 102°C); 0.13 parts of a chiral agent ("LC756" manufactured by BASF Co., Ltd.); 0.035 parts of a photopolymerization initiator (IRGACURE® 379 manufactured by BASF Co., Ltd.); 0.0013 parts of a surfactant ("S242" manufactured by AGC Seimi Chemical Co., Ltd.); and 1.5 parts of cyclopentanone as a solvent.

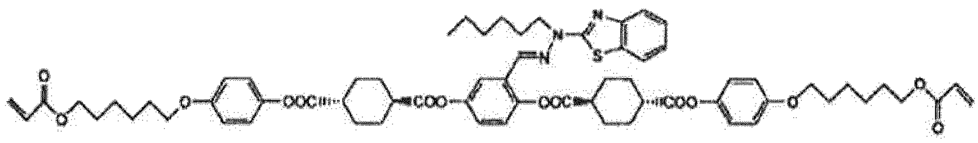

(A)

As a support film, a polyethylene terephthalate film (thickness: 100 μm) of which one surface was subjected to an adhesion facilitating treatment was prepared. A surface of this support film which was not subjected to an adhesion facilitating treatment was subjected to a rubbing treatment. Subsequently, onto the surface having been subjected to a

rubbing treatment, the aforementioned liquid crystal composition was applied. Accordingly, a layer of the liquid crystal composition in an uncured state was formed on one surface of the support film.

Thereafter, drying was performed under the conditions of 80°C and 1 minute to remove the solvent from the layer of the liquid crystal composition. Furthermore, an orientation treatment was performed under the conditions of 130°C and 2 minutes to orient the polymerizable liquid crystal compound.

Subsequently, the layer of the liquid crystal composition was irradiated with UV light at an illuminance of 1000 $mW/cm^2$ and an integrated light quantity of 4000 $mJ/cm^2$ under nitrogen atmosphere, so that the layer of the liquid crystal composition was cured. Thus, a flat cholesteric resin layer with a thickness of 5 μm which does not have a concavo-convex structure was obtained. The half width $W_2$ of the band of light reflection on this cholesteric resin layer was measured. The result was 35 nm. The center wavelength of the reflection was 550 nm.

A specific recognition molecule was immobilized on the resulting cholesteric resin layer that served as the substrate. As the specific recognition molecule, an antibody (anti-human fibrinogen antibody) solution (1 μg/mL) was prepared. This solution was dropped on the surface of the substrate, and left to stand at room temperature for 1 hour. By this operation, physical adsorption of the antibody on the surface by a hydrophobic interaction was achieved, to thereby immobilize the antibody on the surface. Thus, an analysis plate was obtained.

The color tone of the surface of the resulting analysis plate was measured using a spectrometer (USB4000 manufactured by Ocean Optics, Inc. or "TECAN" manufactured by Wako Pure Chemical Industries, Ltd.). As a result, reflection having the peak at a wavelength of 550 nm was observed. Subsequently, an analyte (including human fibrinogen) solution (1 μg/mL) was dropped on the surface, and left to stand at room temperature for 1 hour. The resultant product was washed with ultrapure water, and dried. Then, the color tone on the surface was measured again. As a result, the reflection intensity of the peak at the wavelength of 550 nm decreased by approximately 12% compared to that before dropping the analyte. Thus, a decrease in reflection due to binding to the antigen was clearly observed.

<Reference Example>

From a comparison between the spectra obtained using a spectrometer in Example 1 and Example 2, it was found that the reflection of a portion at a reflectivity of 25% in Example 1 was the band of the light reflection caused by the concavo-convex structure in the cholesteric resin layer having the concavo-convex structure. The calculated half width $W_1$ of the band was 25 nm. The center wavelength of the reflection was 550 nm.

Reference Sign List

10: analysis plate
40: analysis plate
50: analysis plate
60: analysis plate
111: substrate
11B: bottom of surface
11T: top of surface
11U: surface of substrate (concave-convex structure)
121: specific recognition molecule
311: profile of light reflection caused by concavo-convex structure
312: profile of light reflection caused by concavo-convex structure in a state where measurement target substance binds to specific recognition molecule of analysis plate
321: profile of the light reflection caused by the selective reflectivity
410: substrate having a multi-layer structure
411: selective reflection layer
412: support layer
510: substrate having a multi-layer structure
511: selective reflection layer
513: transparent layer having a concavo-convex structure
631: plate-shaped structure
63D: bottom of plate-shaped structure
63H: through hole
L1: line
L2: line

P1: repeating period
P2: repeating period

## Claims

1. An analysis plate (10, 40, 50, 60) comprising: a substrate (111); and a molecule immobilized on a surface (11U) of the substrate (111), the molecule specifically recognizing a measurement target substance,

   wherein the surface (11U) of the substrate (111) has a concavo-convex structure capable of exhibiting a structural color;
   **characterized in that**
   the substrate (111) includes a selective reflection layer in at least part of the layer thereof; wherein the selective reflection layer is a layer which reflects only light within a specific wavelength range and transmits light having other wavelengths due to the molecular structure in the layer, wherein
   a band of light reflection caused by a selective reflectivity of the selective reflection layer (411, 511) falls within a band of light reflection caused by the concavo-convex structure; and
   wherein the band of the light reflection caused by the selective reflectivity has a peak falling within the band of the light reflection caused by the concavo-convex structure.

2. The analysis plate (10, 40, 50, 60) according to claim 1, wherein the selective reflection layer (411, 511) is a resin layer having cholesteric regularity.

3. The analysis plate (10, 40, 50, 60) according to claim 2, wherein the resin layer is obtained by polymerizing the compound of formula (I) in a state in which the compound exhibits the cholesteric phase,

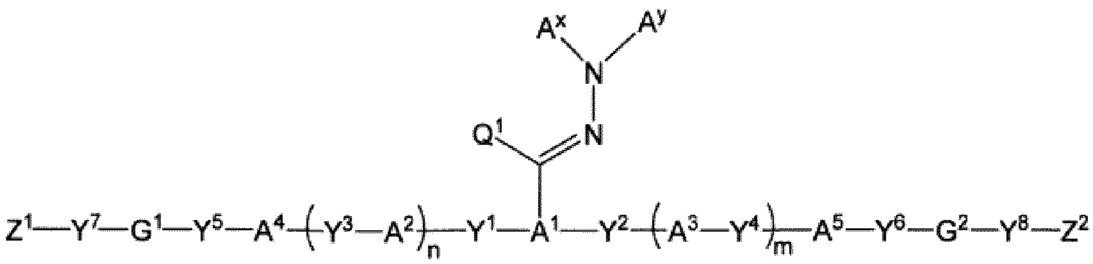

(I)

wherein

   $Y^1$ to $Y^8$ are each independently a chemical single bond, -O-, -S-, -O-C(=O)-, -C(=O)-O-, -O-C(=O)-O-, -$NR^1$-C(=O)-, - C(=O)-$NR^1$-, -O-C(=O)-$NR^1$-, -$NR^1$-C(=O)-O-, -$NR^1$-C(=O)-$NR^1$-, - O-$NR^1$-, or -$NR^1$-O-, wherein $R^1$ is a hydrogen atom or an alkyl group of 1 to 6 carbon atoms,
   $G^1$ and $G^2$ are each independently a divalent aliphatic group of 1 to 20 carbon atoms optionally having a substituent, the aliphatic groups may have one or more per one aliphatic group of -O-, -S-, -O-C(=O)-, -C(=O)-O-, -O-C(=O)-O-, -$NR^2$-C(=O)-, -C(=O)-$NR^2$-, -$NR^2$-, or -C(=O)-inserted therein, provided that cases where two or more -O-or -S- are adjacently inserted are excluded, $R^2$ is a hydrogen atom or an alkyl group of 1 to 6 carbon atoms,
   $Z^1$ and $Z^2$ are each independently an alkenyl group of 2 to 10 carbon atoms that may be unsubstituted or substituted by a halogen atom,
   $A^x$ is an organic group of 2 to 30 carbon atoms having at least one aromatic ring selected from the group consisting of an aromatic hydrocarbon ring and an aromatic heterocyclic ring,
   $A^y$ is a hydrogen atom, an alkyl group of 1 to 20 carbon atoms optionally having a substituent, an alkenyl group of 2 to 20 carbon atoms optionally having a substituent, a cycloalkyl group of 3 to 12 carbon atoms optionally having a substituent, an alkynyl group of 2 to 20 carbon atoms optionally having a substituent, -C(=O)-$R^3$, -$SO_2$-$R^4$, -C(=S)NH-$R^9$, or an organic group of 2 to 30 carbon atoms having at least one aromatic ring selected from the group consisting of an aromatic hydrocarbon ring and an aromatic heterocyclic ring, wherein $R^3$ is an alkyl group of 1 to 20 carbon atoms optionally having a substituent, an alkenyl group of 2 to 20 carbon atoms

optionally having a substituent, a cycloalkyl group of 3 to 12 carbon atoms optionally having a substituent, or an aromatic hydrocarbon group of 5 to 12 carbon atoms, $R^4$ is an alkyl group of 1 to 20 carbon atoms, an alkenyl group of 2 to 20 carbon atoms, a phenyl group, or a 4-methylphenyl group, $R^9$ is an alkyl group of 1 to 20 carbon atoms optionally having a substituent, an alkenyl group of 2 to 20 carbon atoms optionally having a substituent, a cycloalkyl group of 3 to 12 carbon atoms optionally having a substituent, or an aromatic group of 5 to 20 carbon atoms optionally having a substituent, and the aromatic ring that $A^x$ and $A^y$ have may have a substituent, and $A^x$ and $A^y$ may form a ring together,
$A^1$ is a trivalent aromatic group optionally having a substituent,
$A^2$ and $A^3$ are each independently a divalent alicyclic hydrocarbon group of 3 to 30 carbon atoms optionally having a substituent,
$A^4$ and $A^5$ are each independently a divalent aromatic group of 6 to 30 carbon atoms optionally having a substituent,
$Q^1$ is a hydrogen atom or an alkyl group of 1 to 6 carbon atoms optionally having a substituent,
m and n are each independently 0 or 1.

**4.** The analysis plate (10, 40, 50, 60) according to claim 2 or claim 3, wherein the half width of the selective reflection band of the cholesteric resin layer is 80 nm or less.

**5.** The analysis plate (10, 40, 50, 60) according to any one of claims 1 to 4, wherein a layer positioned at the surface (11U) of the substrate (111) is the selective reflection layer (411, 511).

**6.** An analysis method for measuring a measurement target substance contained in an analyte, comprising the steps of:

preparing the analysis plate (10, 40, 50, 60) according to any one of claims 1 to 5 which includes, as the molecule specifically recognizing the measurement target substance, a molecule that specifically binds to the measurement target substance;
bringing the analyte into contact with the analysis plate (10, 40, 50, 60); and
measuring a change in light reflection amount of the surface of the analysis plate (10, 40, 50, 60) caused by the contact with the analyte.

**7.** A method for producing the analysis plate (10, 40, 50, 60) according to any one of claims 1 to 5, comprising the steps of:

forming a layer of a liquid crystal composition containing a polymerizable liquid crystal compound capable of exhibiting a cholesteric liquid crystal phase;
semi-curing the layer of the liquid crystal composition to obtain a semi-cured layer;
forming a concavo-convex structure capable of exhibiting a structural color on a surface of the semi-cured layer;
fully-curing the semi-cured layer to obtain a substrate (111), wherein the substrate (111) includes a selective reflection layer in at least part of the layer thereof; and
immobilizing a molecule that specifically recognizes a measurement target substance on the surface (11U) of the substrate (111).

## Patentansprüche

**1.** Analyseplatte (10, 40, 50, 60) umfassend: ein Substrat (111); und ein Molekül, das auf einer Oberfläche (11U) des Substrats (111) immobilisiert ist, wobei das Molekül spezifisch eine Messzielsubstanz erkennt,
wobei die Oberfläche (11U) des Substrats (111) eine konkav-konvexe Struktur aufweist, die in der Lage ist, eine Strukturfarbe zu zeigen;
**dadurch gekennzeichnet, dass**

das Substrat (111) eine selektive Reflexionsschicht in mindestens einem Teil seiner Schicht enthält; wobei die selektive Reflexionsschicht eine Schicht ist, die nur Licht innerhalb eines bestimmten Wellenlängenbereichs reflektiert und Licht mit anderen Wellenlängen aufgrund der Molekularstruktur in der Schicht durchlässt, wobei ein Band der Lichtreflexion, das durch ein selektives Reflexionsvermögen der selektiven Reflexionsschicht (411, 511) verursacht wird, in ein Band der durch die konkav-konvexe Struktur verursachten Lichtreflexion fällt; und wobei das Band der durch das selektive Reflexionsvermögen verursachten Lichtreflexion einen Peak aufweist, der in das Band der durch die konkav-konvexe Struktur verursachten Lichtreflexion fällt.

**2.** Analyseplatte (10, 40, 50, 60) gemäß Anspruch 1, wobei die selektive Reflexionsschicht (411, 511) eine Harzschicht mit cholesterischer Regelmäßigkeit ist.

**3.** Analyseplatte (10, 40, 50, 60) gemäß Anspruch 2, wobei die Harzschicht durch Polymerisation der Verbindung der Formel (I) in einem Zustand, in dem die Verbindung die cholesterische Phase aufweist, erhalten wird,

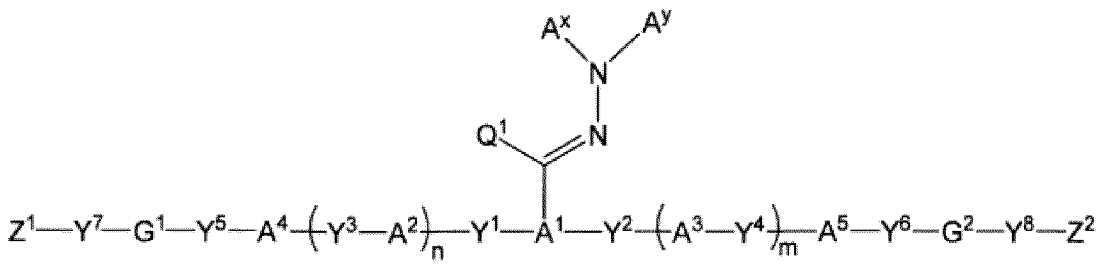

(I)

wobei

$Y^1$ bis $Y^8$ jeweils unabhängig voneinander eine chemische Einfachbindung, -O-, -S-, -O-C(=O)-, -C(=O)-O-, -O-C(=O)-O-, -$NR^1$-C(=O)-, -C(=O)-$NR^1$-, -O-C(=O)-$NR^1$-, -$NR^1$-C(=O)-O-, -$NR^1$-C(=O)-$NR^1$-, -O-$NR^1$ -, oder -$NR^1$ -O- sind, wobei $R^1$ ein Wasserstoffatom oder eine Alkylgruppe mit 1 bis 6 Kohlenstoffatomen ist,
$G^1$ und $G^2$ jeweils unabhängig voneinander eine divalente aliphatische Gruppe mit 1 bis 20 Kohlenstoffatomen sind, die gegebenenfalls einen Substituenten aufweist, wobei die aliphatischen Gruppen pro aliphatischer Gruppe eine oder mehrere der Gruppen -O-, -S-, -O-C(=O)-, -C(=O)-O-, -O-C(=O)-O-, -$NR^2$-C(=O)-, -C(=O)-$NR^2$-, -$NR^2$ - oder -C(=O)- darin eingefügt aufweisen können, mit der Maßgabe, dass Fälle, in denen zwei oder mehr -O- oder -S- nebeneinander eingefügt sind, ausgeschlossen sind, $R^2$ ein Wasserstoffatom oder eine Alkylgruppe mit 1 bis 6 Kohlenstoffatomen ist,
$Z^1$ und $Z^2$ jeweils unabhängig voneinander eine Alkenylgruppe mit 2 bis 10 Kohlenstoffatomen sind, die unsubstituiert oder durch ein Halogenatom substituiert sein kann,

$A^x$ eine organische Gruppe mit 2 bis 30 Kohlenstoffatomen und mindestens einem aromatischen Ring ausgewählt aus der Gruppe bestehend aus einem aromatischen Kohlenwasserstoffring und einem aromatischen heterocyclischen Ring ist,
$A^y$ ein Wasserstoffatom, eine Alkylgruppe mit 1 bis 20 Kohlenstoffatomen, die gegebenenfalls einen Substituenten aufweist, eine Alkenylgruppe mit 2 bis 20 Kohlenstoffatomen, die gegebenenfalls einen Substituenten aufweist, eine Cycloalkylgruppe mit 3 bis 12 Kohlenstoffatomen, die gegebenenfalls einen Substituenten aufweist, eine Alkynylgruppe mit 2 bis 20 Kohlenstoffatomen, die gegebenenfalls einen Substituenten
aufweist, -C(=O)-$R^3$ , -$SO_2$ -$R^4$ , -C(=S)NH-$R^9$ , oder eine organische Gruppe mit 2 bis 30 Kohlenstoffatomen mit mindestens einem aromatischen Ring, ausgewählt aus der Gruppe bestehend aus einem aromatischen Kohlenwasserstoffring und einem aromatischen heterocyclischen Ring, ist, wobei $R^3$ eine Alkylgruppe mit 1 bis 20 Kohlenstoffatomen, die gegebenenfalls einen Substituenten aufweist, eine Alkenylgruppe mit 2 bis 20 Kohlenstoffatomen, die gegebenenfalls einen Substituenten aufweist eine Cycloalkylgruppe mit 3 bis 12 Kohlenstoffatomen, die gegebenenfalls einen Substituenten aufweist, oder eine aromatische Kohlenwasserstoffgruppe mit 5 bis 12 Kohlenstoffatomen ist, $R^4$ eine Alkylgruppe mit 1 bis 20 Kohlenstoffatomen, eine Alkenylgruppe mit 2 bis 20 Kohlenstoffatomen, eine Phenylgruppe oder eine 4-Methylphenylgruppe ist, $R^9$ eine Alkylgruppe mit 1 bis 20 Kohlenstoffatomen ist, die gegebenenfalls einen Substituenten aufweist, eine Alkenylgruppe mit 2 bis 20 Kohlenstoffatomen, die gegebenenfalls einen Substituenten aufweist, eine Cycloalkylgruppe mit 3 bis 12 Kohlenstoffatomen, die gegebenenfalls einen Substituenten aufweist, oder eine aromatische Gruppe mit 5 bis 20 Kohlenstoffatomen, die gegebenenfalls einen Substituenten aufweist, und der aromatische Ring, den $A^x$ und $A^y$ aufweisen, einen Substituenten aufweisen kann, und $A^x$ und $A^y$ zusammen einen Ring bilden können,

$A^1$ eine trivalente aromatische Gruppe ist, die gegebenenfalls einen Substituenten aufweist,
$A^2$ und $A^3$ jeweils unabhängig voneinander eine divalente alicyclische Kohlenwasserstoffgruppe mit 3 bis 30 Kohlenstoffatomen sind, die gegebenenfalls einen Substituenten aufweist,
$A^4$ und $A^5$ jeweils unabhängig voneinander eine divalente aromatische Gruppe mit 6 bis 30 Kohlenstoffatomen sind, die gegebenenfalls einen Substituenten aufweist,

$Q^1$ ein Wasserstoffatom oder eine Alkylgruppe mit 1 bis 6 Kohlenstoffatomen ist, die gegebenenfalls einen Substituenten aufweist,
m und n jeweils unabhängig voneinander 0 oder 1 sind.

**4.** Analyseplatte (10, 40, 50, 60) gemäß Anspruch 2 oder Anspruch 3, wobei die Halbwertsbreite des selektiven Reflexionsbands der cholesterischen Harzschicht 80 nm oder weniger beträgt.

**5.** Analyseplatte (10, 40, 50, 60) gemäß einem der Ansprüche 1 bis 4, wobei eine an der Oberfläche (11U) des Substrats (111) angeordnete Schicht die selektive Reflexionsschicht (411, 511) ist.

**6.** Analyseverfahren zur Messung einer in einem Analyten enthaltenen Messzielsubstanz, das die folgenden Schritte umfasst:

Herstellung der Analyseplatte (10, 40, 50, 60) gemäß einem der Ansprüche 1 bis 5, die als das Molekül, das die Messzielsubstanz spezifisch erkennt, ein Molekül enthält, das spezifisch an die Messzielsubstanz bindet;
Inkontaktbringen des Analyten mit der Analyseplatte (10, 40, 50, 60); und
Messung einer Änderung des Umfangs der Lichtreflexion der Oberfläche der Analyseplatte (10, 40, 50, 60), die durch den Kontakt mit dem Analyten verursacht wird.

**7.** Verfahren zur Herstellung der Analyseplatte (10, 40, 50, 60) gemäß einem der Ansprüche 1 bis 5, umfassend die folgenden Schritte:

Bilden einer Schicht aus einer Flüssigkristallzusammensetzung, die eine polymerisierbare Flüssigkristallverbindung enthält, die in der Lage ist, eine cholesterische Flüssigkristallphase zu zeigen;
Halbhärten der Schicht aus der Flüssigkristallzusammensetzung, um eine halbgehärtete Schicht zu erhalten;
Bilden einer konkav-konvexen Struktur, die in der Lage ist, eine Strukturfarbe auf einer Oberfläche der halbgehärteten Schicht zu zeigen;
vollständiges Aushärten der halbgehärteten Schicht, um ein Substrat (111) zu erhalten, wobei das Substrat (111) eine selektive Reflexionsschicht in mindestens einem Teil seiner Schicht enthält; und
Immobilisieren eines Moleküls, das eine Messzielsubstanz spezifisch erkennt, auf der Oberfläche (11U) des Substrats (111).

## Revendications

**1.** Plaque d'analyse (10, 40, 50, 60) comprenant : un substrat (111) ; et une molécule immobilisée sur une surface (11U) du substrat (111), la molécule reconnaissant spécifiquement une substance cible de mesure,
dans laquelle

la surface (11U) du substrat (111) a une structure concavo-convexe capable de présenter une couleur structurelle ;
**caractérisée en ce que**
le substrat (111) comprend une couche de réflexion sélective dans au moins une partie de sa couche ; dans laquelle la couche de réflexion sélective est une couche qui réfléchit uniquement la lumière dans une plage de longueurs d'onde spécifique et émet une lumière ayant d'autres longueurs d'onde en raison de la structure moléculaire de la couche, dans laquelle
une bande de réflexion de la lumière provoquée par une réflectivité sélective de la couche de réflexion sélective (411, 511) tombe dans une bande de réflexion de la lumière provoquée par la structure concavo-convexe ; et dans laquelle la bande de la réflexion de la lumière provoquée par la réflectivité sélective a un pic tombant dans la bande de la réflexion de la lumière provoquée par la structure concavo-convexe.

**2.** Plaque d'analyse (10, 40, 50, 60) selon la revendication 1, dans laquelle la couche de réflexion sélective (411, 511) est une couche de résine ayant une régularité cholestérique.

**3.** Plaque d'analyse (10, 40, 50, 60) selon la revendication 2, dans laquelle la couche de résine est obtenue par polymérisation du composé de formule (I) dans un état dans lequel le composé présente la phase cholestérique,

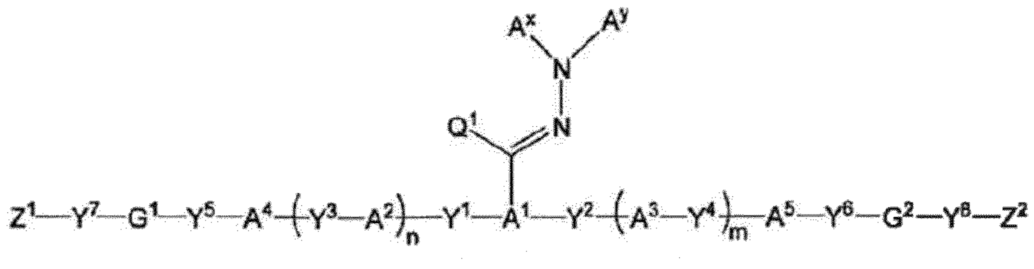

(I)

dans laquelle

$Y^1$ à $Y^8$ sont chacun indépendamment une liaison simple chimique, -O-, -S-, -O-C(=O)-, -C(=O)-O-, -O-C(=O)-O-, - $NR^1$-C(=O)-, -C(=O)-$NR^1$-, -O-C(=O)-$NR^1$-, -$NR^1$-C(=O)-O-, - $NR^1$-C(=O)-$NR^1$- , -O-$NR^1$- ou -$NR^1$-O-, dans laquelle $R^1$ est un atome d'hydrogène ou un groupe alkyle de 1 à 6 atomes de carbone,
$G^1$ et $G^2$ sont chacun indépendamment un groupe aliphatique divalent de 1 à 20 atomes de carbone ayant éventuellement un substituant, les groupes aliphatiques pouvant avoir un ou plusieurs par groupe aliphatique de - O-, -S-, -O-C(=O)-, -C(=O)-O-, -O-C(=O)-O-, -$NR^2$-C(=O)-, - C(=O)-$NR^2$-, -$NR^2$- ou -C(=O)- insérés dans ceux-ci, à condition que les cas où deux -O- ou -S- ou plus sont insérés de manière adjacente soient exclus, $R^2$ est un atome d'hydrogène ou un groupe alkyle de 1 à 6 atomes de carbone,
$Z^1$ et $Z^2$ sont chacun indépendamment un groupe alcényle de 2 à 10 atomes de carbone qui peut être non substitué ou substitué par un atome d'halogène,
$A^x$ est un groupe organique de 2 à 30 atomes de carbone ayant au moins un cycle aromatique choisi dans le groupe constitué par un cycle hydrocarboné aromatique et un cycle hétérocyclique aromatique,
$A^y$ est un atome d'hydrogène, un groupe alkyle de 1 à 20 atomes de carbone ayant éventuellement un substituant, un groupe alcényle de 2 à 20 atomes de carbone ayant éventuellement un substituant, un groupe cycloalkyle de 3 à 12 atomes de carbone ayant éventuellement un substituant, un groupe alcynyle de 2 à 20 atomes de carbone ayant éventuellement un substituant, -C(=O)-$R^3$, -$SO_2$-$R^4$,-C(=S)NH-$R^9$ ou un groupe organique de 2 à 30 atomes de carbone ayant au moins un cycle aromatique choisi dans le groupe constitué par un cycle hydrocarboné aromatique et un cycle hétérocyclique aromatique, dans laquelle, $R^3$ est un groupe alkyle de 1 à 20 atomes de carbone ayant éventuellement un substituant, un groupe alcényle de 2 à 20 atomes de carbone ayant éventuellement un substituant, un groupe cycloalkyle de 3 à 12 atomes de carbone ayant éventuellement un substituant ou un groupe hydrocarboné aromatique de 5 à 12 atomes de carbone, $R^4$ est un groupe alkyle de 1 à 20 atomes de carbone, un groupe alcényle de 2 à 20 atomes de carbone, un groupe phényle ou un groupe 4-méthylphényle, $R^9$ est un groupe alkyle de 1 à 20 atomes de carbone ayant éventuellement un substituant, un groupe alcényle de 2 à 20 atomes de carbone ayant éventuellement un substituant, un groupe cycloalkyle de 3 à 12 atomes de carbone ayant éventuellement un substituant ou un groupe aromatique de 5 à 20 atomes de carbone ayant éventuellement un substituant, et le cycle aromatique que $A^x$ et $A^y$ ont peut avoir un substituant, et $A^x$ et $A^y$ peuvent former conjointement un cycle,
$A^1$ est un groupe aromatique trivalent ayant éventuellement un substituant,
$A^2$ et $A^3$ sont chacun indépendamment un groupe hydrocarboné alicyclique divalent de 3 à 30 atomes de carbone ayant éventuellement un substituant,
$A^4$ et $A^5$ sont chacun indépendamment un groupe aromatique divalent de 6 à 30 atomes de carbone ayant éventuellement un substituant,
$Q^1$ est un atome d'hydrogène ou un groupe alkyle de 1 à 6 atomes de carbone ayant éventuellement un substituant,
m et n sont chacun indépendamment 0 ou 1.

**4.** Plaque d'analyse (10, 40, 50, 60) selon la revendication 2 ou la revendication 3, dans laquelle la demi-largeur de la bande de réflexion de la lumière de la couche de résine cholestérique est inférieure ou égale à 80 nm.

**5.** Plaque d'analyse (10, 40, 50, 60) selon l'une quelconque des revendications 1 à 4, dans laquelle une couche positionnée au niveau de la surface (11U) du substrat (111) est la couche de réflexion sélective (411, 511) .

**6.** Procédé d'analyse pour mesurer une substance cible de mesure contenue dans un analyte, comprenant les étapes consistant à :

préparer la plaque d'analyse (10, 40, 50, 60) selon l'une quelconque des revendications 1 à 5 qui comprend, en tant que molécule reconnaissant spécifiquement la substance cible de mesure, une molécule qui se lie spécifiquement à la substance cible de mesure ;
mettre en contact l'analyte avec la plaque d'analyse (10, 40, 50, 60) ; et
mesurer un changement de la quantité de réflexion de la lumière de la surface de la plaque d'analyse (10, 40, 50, 60) provoquée par la mise en contact avec l'analyte.

**7.** Procédé de production de la plaque d'analyse (10, 40, 50, 60) selon l'une quelconque des revendications 1 à 5, comprenant les étapes consistant à :

former une couche d'une composition de cristaux liquides contenant un composé à cristaux liquides polymérisable capable de présenter une phase de cristaux liquides cholestérique ;
semi-durcir la couche de la composition de cristaux liquides pour obtenir une couche semi-durcie ;
former une structure concavo-convexe capable de présenter une couleur structurelle sur une surface de la couche semi-durcie ;
durcir complètement la couche semi-durcie pour obtenir un substrat (111), le substrat (111) comprenant une couche de réflexion de la lumière dans au moins une partie de sa couche ; et
immobiliser une molécule qui reconnaît spécifiquement une substance cible de mesure sur la surface (11U) du substrat (111).

FIG.1

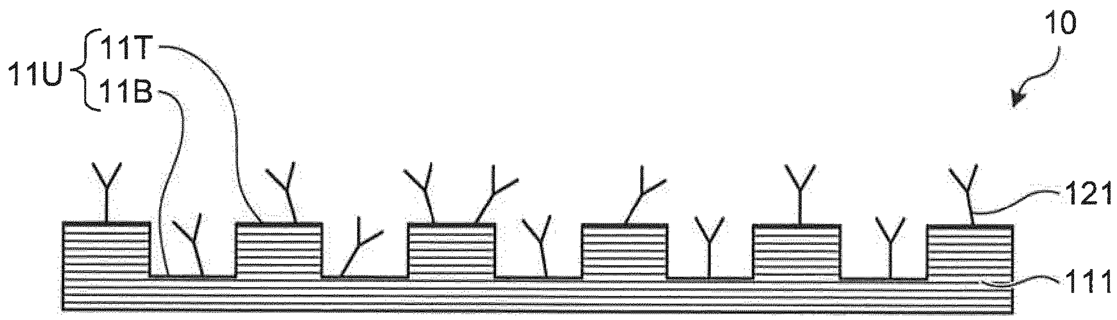


FIG.2

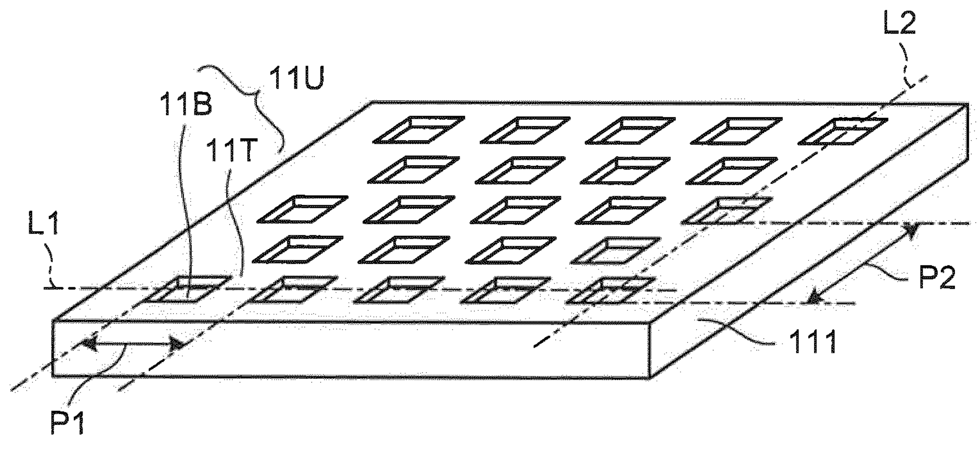

FIG.3

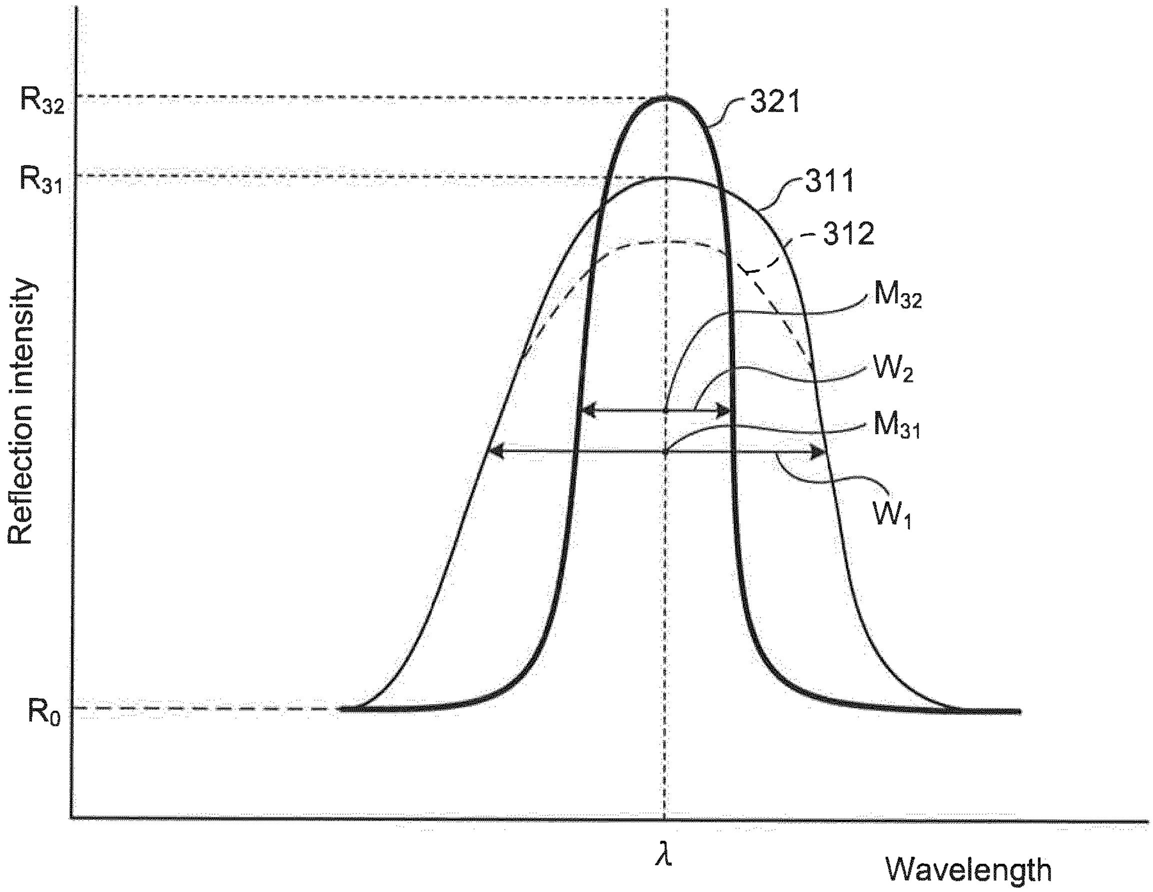

R32
R31
R0
Reflection intensity
321
311
312
M32
W2
M31
W1
λ
Wavelength

FIG.4

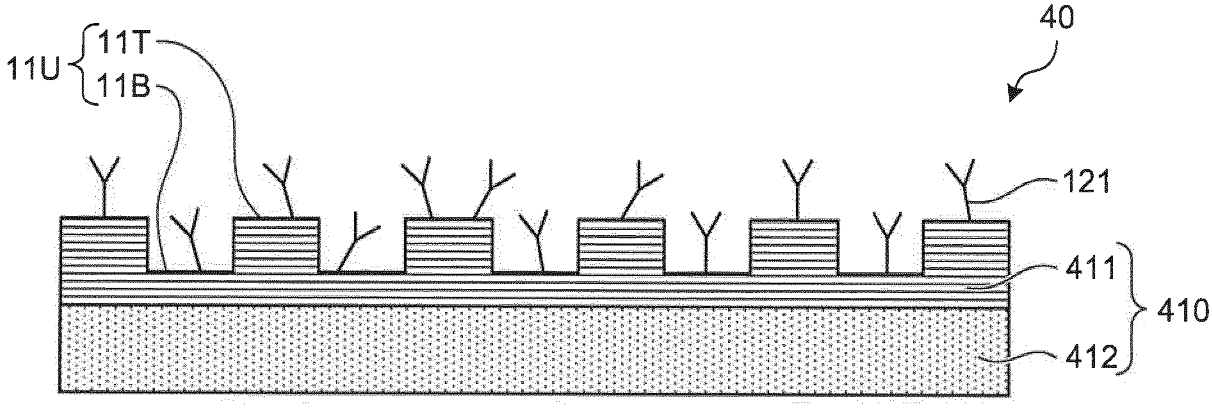


FIG.5

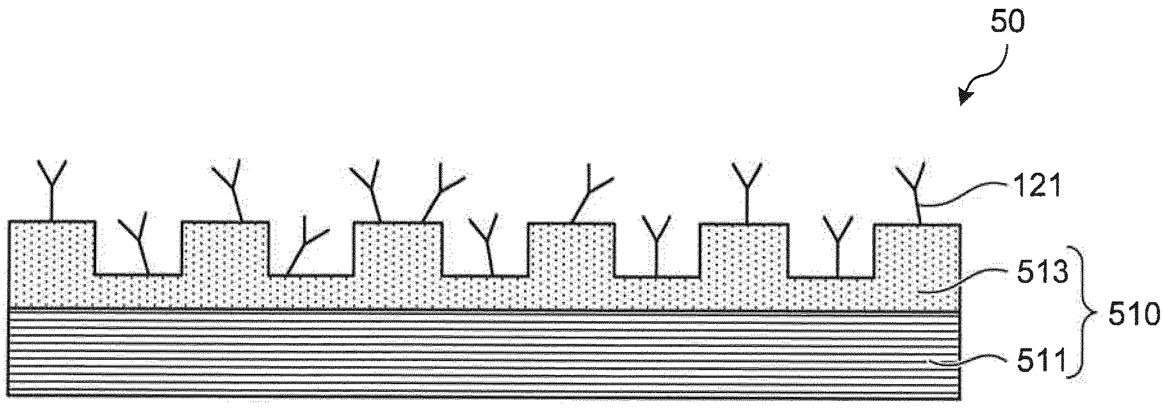

FIG.6

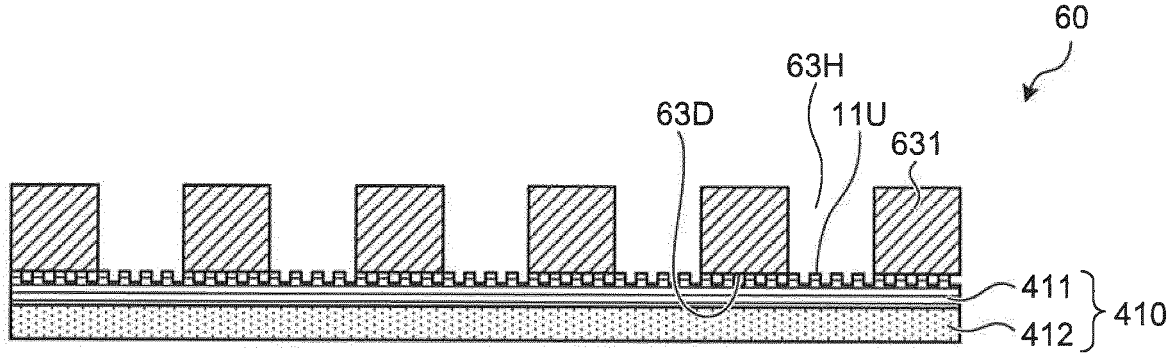

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2011080848 A
- JP 2010197046 A
- US 20030017580 A1
- US 20060180750 A1
- WO 2014069515 A1
- WO 2015064581 A1
- WO 2012147904 A1
- JP 2009300662 A
- JP 2003066214 A
- JP 2003313187 A
- US 6468444 B1
- WO 9800428 A1
- US 3046127 A
- US 2951758 A
- US 4212970 A
- US 2367661 A
- US 2367670 A
- US 2448828 A
- US 2722512 A
- US 3549367 A
- JP SHO60105667 A
- US 4239850 A